(19) Europäisches Patentamt
European Patent Office
Office européen des brevets

(11) Publication number: 0 107 165 B1

# (12) EUROPEAN PATENT SPECIFICATION

(45) Date of publication of patent specification: 15.04.87

(51) Int. Cl.$^4$: C 07 D 487/14, A 61 K 31/52 // (C07D487/14, 239:00, 239:00, 235:00)

(21) Application number: 83110335.3

(22) Date of filing: 17.10.83

## (54) Substituted 9H-8-oxo-pyrimido(2,1-f)purine-2,4-diones, process for their production and pharmaceutical compositions containing them.

(30) Priority: 25.10.82 US 436247

(43) Date of publication of application: 02.05.84 Bulletin 84/18

(45) Publication of the grant of the patent: 15.04.87 Bulletin 87/16

(84) Designated Contracting States: AT BE CH DE FR GB IT LI LU NL SE

(58) References cited:
AT-B- 361 503
DE-A-1 809 013

(73) Proprietor: SCHERING CORPORATION
2000 Galloping Hill Road
Kenilworth, New Jersey 07033 (US)

(72) Inventor: Blythin, David John
487 Mountain Avenue
North Caldwell New Jersey 07006 (US)

(74) Representative: von Kreisler, Alek et al
Patentanwälte Von Kreisler-Schönwald-Fues-Keller Selting-Werner Deichmannhaus am Hauptbahnhof
D-5000 Köln 1 (DE)

EP 0 107 165 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to substituted 9*H*-8-oxo-pyrimido[2,1-f]purine-2,4-diones and tautomers thereof and their salts. These compounds are useful as anti-inflammatory agents for treating inflammatory conditions such as arthritis, spondylitis, and tendonitis in mammals.

Tetrahydropyrimido[2,1-f]theophyllines useful in cardiotherapy are known from AT—B—361 503.

Pyrimido[2,1-f]purines, useful for a variety of biological and chemotherapeutic purposes, including treatment of certain inflammatory conditions, are known from US—A—3,637,684 and 3,770,741 and DE—A—1,809,013.

This invention provides a compound of the general formula (I)

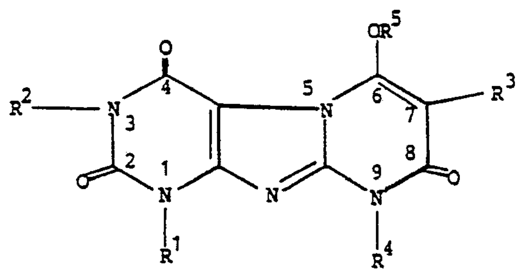

(I)

and its tautomer and the pharmaceutically acceptable salts thereof,

wherein

$R^1$ and $R^2$ are the same or different and are hydrogen, alkyl having 1 to 8 carbon atoms, cycloalkyl having 3 to 8 carbon atoms, alkenyl having 3 to 8 carbon atoms, alkynyl having 3 to 8 carbon atoms, aryl such as phenyl, naphthyl, phenanthryl, or substituted phenyl;

alkyl (having 1 to 8 carbon atoms) substituted by cycloalkyl (having 3 to 8 carbon atoms), aryl as defined above or heterocyclic radical selected from quinolinyl, isoquinolinyl, pyridinyl, thiazolyl, 1,3,4-thiadiazolyl, and thiophenyl;

each of the aforementioned substituted phenyl and substituted heterocyclic radicals may be substituted with one to three radicals that are independently selected from halogen (i.e., fluoro, chloro, bromo, iodo), trifluoromethyl, nitro, cyano, $C_1$ to $C_6$ alkoxy, $C_1$ to $C_6$ alkyl, phenyl, hydroxy, $C_1$ to $C_6$ alkylthio,

$$-\overset{O^-}{\overset{|}{S^+}}-(C_1 \text{ to } C_6 \text{ alkyl}),$$

$-SO_2-(C_1$ to $C_6$ alkyl), $-O-(C_3$ to $C_8$ alkenyl), $-O-(C_3$ to $C_8$ alkynyl), $-OC_mH_{2m}$-phenyl wherein m is an integer from 0 to 4, $C_1$ to $C_6$ acyloxy, hydroxy-($C_1$ to $C_6$ alkyl), $C_1$ to $C_6$ acyloxy)-($C_1$ to $C_6$ alkyl)—, cyano-($C_1$ to $C_6$ alkyl), $-CONH_2$, $-CO_2H$, and $-(C_1$ to $C_4$ alkyl)-$CO_2(C_1$ to $C_4$ alkyl);

$R^3$ is hydrogen, alkyl containing 1 to 8 carbon atoms, cycloalkyl containing 3 to 8 carbon atoms, alkenyl containing 2 to 8 carbon atoms, cycloalkenyl containing 5 to 8 carbon atoms, alkynyl containing 3 to 8 carbon atoms, acyloxyalkyl containing 2 to 12 carbon atoms, oxoalkyl containing 1 to 8 carbon atoms, aryl as defined above;

alkyl (containing 1 to 8 carbon atoms) substituted by cycloalkyl (containing 3 to 8 carbon atoms), cycloalkenyl (containing 5 to 8 carbon atoms), aryl as defined above, hydroxy, cyano, halo or heterocyclic radicals as defined above;

-alkyl—X—$C_nH_{2n+1}$ wherein alkyl contains 1 to 8 carbon atoms, n is an integer from 0 to 4 and X is O,S,$S^+-O^-$, $SO_2$ or $-N-C_pH_{2p+1}$ wherein p is an integer from 0 to 4;

$-(CH_2)_q$ C(O)$NR^6R^7$ (wherein $R^6$ and $R^7$ are independently hydrogen or alkyl having from 1 to 8 carbon atoms, and q is an integer from 0 to 6), $-(CH_2)_r C(O)OR^8$ (wherein $R^8$ is hydrogen or alkyl having from 1 to 8 carbon atoms and r is an integer from 0 to 6);

$R^4$ is hydrogen, alkyl containing 1 to 8 carbon atoms, aryl as defined above, a heterocyclic radical as defined above;

alkyl (containing 1 to 8 carbon atoms) substituted by cycloalkyl (containing 3 to 8 carbon atoms), aryl as defined above or a heterocyclic radical as defined above;

$R^5$ is hydrogen, acyl containing 2 to 8 carbon atoms or alkyl containing 1 to 4 carbon atoms.

The following terms, unless specified otherwise, are understood to be defined as follows:

The alkyl groups or alkyl portions of other groups may be a straight chain, a branched chain or combinations thereof. The term alkyl includes methyl, ethyl, propyl, butyl, pentyl, hexyl, heptyl and octyl and isomers thereof such as isopropyl, tert.butyl, neopentyl, dimethylbutyl and the like. Cycloalkyl means groups such as cyclopropyl, cyclobutyl, cyclopentyl, cyclohexyl, cycloheptyl and cyclooctyl. The terms alkenyl, alkynyl and cycloalkenyl refer to analogous unsaturated groups.

The term acyl refers to radicals derived form organic acids by the removal of the hydroxyl group. Preferably the acyl groups are derived from alkanoic acids. In the group acyloxyalkyl ($R^3$) the acyl group is preferably derived from an alkanoic acid containing 2 to 5 carbon atoms, preferably from acetic acid. Aryl is phenyl, naphthyl, phenanthryl, or substituted phenyl, wherein the possible substituents are set forth below.

Heterocyclic, as used herein, refers to a substituted or unsubstituted heterocyclic radical selected from quinolinyl, isoquinolinyl, pyridinyl, thiazolyl, 1,3,4-thiadiazolyl, and thiophenyl.

Each of the aforementioned substituted phenyl and substituted heterocyclic radicals may be substituted with one to three radicals that are independently selected from halogen (i.e., fluoro, chloro, bromo, iodo), trifluoromethyl, nitro, cyano, $C_1$ to $C_6$ alkoxy, $C_1$ to $C_6$ alkyl, phenyl, hydroxy, $C_1$ to $C_6$ alkylthio,

$$\overset{O^-}{\underset{|}{}}\\ -S^+-(C_1 \text{ to } C_6 \text{ alkyl}),$$

$-SO_2-(C_1$ to $C_6$ alkyl), $-O-(C_3$ to $C_8$ alkenyl), $-O-(C_3$ to $C_8$ alkynyl), $-OC_mH_{2m}$-phenyl wherein m is an integer from 0 to 4, $C_1$ to $C_6$ acyloxy, hydroxy-($C_1$ to $C_6$ alkyl), $C_1$ to $C_6$ acyloxy)-($C_1$ to $C_6$ alkyl)—, cyano-($C_1$ to $C_6$ alkyl), $-CONH_2$, $-CO_2H$, and $-(C_1$ to $C_4$ alkyl)-$CO_2(C_1$ to $C_4$ alkyl).

A preferred embodiment of the present invention relates to compounds of the formula I and the pharmaceutically acceptable salts thereof, wherein

$R^1$ and $R^2$ are the same or different and are alkyl having 1 to 8 carbon atoms, cycloalkyl having 3 to 8 carbon atoms, alkenyl having 3 to 8 carbon atoms, phenyl;

alkyl (having 1 to 8 carbon atoms) substituted by cycloalkyl (having 3 to 8 carbon atoms), phenyl, pyridyl or thienyl;

$R^3$ is hydrogen, alkyl containing 1 to 8 carbon atoms, cycloalkyl containing 3 to 8 carbon atoms, alkenyl containing 2 to 8 carbon atoms, cycloalkenyl containing 5 to 8 carbon atoms, alkynyl containing 3 to 8 carbon atoms, acyloxyalkyl containing 2 to 12 carbon atoms, phenyl;

alkyl (containing 1 to 8 carbon atoms) substituted by phenyl, hydroxy, cyano, halo, pyridyl or thienyl;

-alkyl-X—$C_nH_{2n+1}$ wherein alkyl contains 1 to 8 carbon atoms, n is an integer from 0 to 4 and X is O, S or —N—$C_pH_{2p+1}$ wherein p is an integer from 0 to 4;

$-(CH_2)_rC(O)OR^8$ (wherein $R^8$ is hydrogen or alkyl having from 1 to 8 carbon atoms and r is an integer from 0 to 6);

$R^4$ is alkyl containing 1 to 8 carbon atoms, phenyl, pyridyl or thienyl;

alkyl (containing 1 to 8 carbon atoms) substituted by cycloalkyl (containing 3 to 8 carbon atoms), phenyl, pyridyl or thienyl;

$R^5$ is hydrogen, acyl containing 2 to 8 carbon atoms or alkyl containing 1 to 4 carbon atoms.

A more preferred embodiment of the present invention relates to compounds of the formula I and the pharmaceutically acceptable salts thereof, wherein

$R^1$ and $R^2$ are the same or different and are alkyl having 1 to 4 carbon atoms;

$R^3$ is hydrogen, alkyl containing 1 to 5 carbon atoms, alkenyl containing 3 to 5 carbon atoms, cyclohexenyl, alkynyl containing 3 carbon atoms, acyloxyalkyl containing 4 carbon atoms, phenyl;

alkyl (containing 1 or 2 carbon atoms) substituted by phenyl, hydroxy or cyano;

$$-CH_2-CH_2-N-(CH_3)_2,\ -CH_2C(O)OH,\ -CH_2C(O)OCH_3,\ -CH_2CH_2OCH_2CH_3,$$
$$-CH_2CH_2SCH_2CH_3 \text{ or } -CO_2C_2H_5;$$

$R^4$ is alkyl containing 1 to 6 carbon atoms, phenyl or fluoro-substituted phenyl;

alkyl (containing 1 or 2 carbon atoms) substituted by cyclohexyl, phenyl (unsubstituted or substituted by fluoro, chloro, or methoxy), thienyl or pyridyl;

$R^5$ is hydrogen, acetyl or methyl.

An embodiment of particular importance are compounds of formula I and the pharmaceutically acceptable salts thereof, wherein $R^1$ and $R^2$ are as defined above, $R^3$ is n-propyl, n-butyl, or 3-methyl-2-butenyl, $R^4$ is benzyl or p-fluorobenzyl and $R^5$ is hydrogen, especially those wherein $R^1$ and $R^2$ are methyl.

As the compounds of the present invention wherein $R^5$ is hydrogen are acidic in character they will form pharmaceutically acceptable metal or amine salts. Illustrative examples of such metals are alkali metals such as lithium , sodium and potassium and alkaline earth metals such as magnesium and calcium. Other metal salts, for example, the aluminum, zinc and iron and copper salts, are also within the scope of this invention. Generally, the sodium salts are preferred, (i.e., compounds (I) wherein $R^5$ is replaced by $Na^+$). Illustrative of the amines are those derived from primary, secondary or tertiary amines. Examples of suitable amines are methylamine, dimethylamine, triethylamine, ethylamine, dibutylamine, triisopropylamine, *N*-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, diethylenetriamine and like aliphatic, cycloaliphatic and araliphatic amines containing up to and including about 18 carbon atoms, as well as heterocyclic amines, for example,

piperidine, morpholine, pyrrolidine, piperazine and lower alkyl derivatives thereof, such as 1-methylpiperidine, 4-ethylmorpholine, 1-isopropylpyrrolidine, 2-methylpyrrolidine, 1,4-dimethylpiperazine and 2-methylpiperidine, as well as amines containing water solubilizing or hydrophilic groups, for example, mono-, di- and triethanolamine, ethyldiethylanolamine, *n*-butylethanolamine, 2-amino-1-butanol, 2-amino-2-ethyl-1,3-propanediol, 2-amino-2-methyl-1-propanol, tris(hydroxymethyl)aminomethane, *N*-phenylethanolamine, *N*-(*p*-tetramylphenyl)diethanolamine, galactamine, *N*-methylglucamine and *N*-methylglucosamine. Preferred amines are ethylenediamine, 1-methylpiperidine, 4-ethylmorpholine, mono-, di-, and triethanolamine, ethyldiethylanolamine, 2-amino-2-ethyl-1,3-propanediol, tris(hydroxymethyl)aminomethane, galactamine, *N*-methylglucamine and *N*-methylglucosamine. More preferred amines are ethylenediamine, mono-, di-, and triethanolamine, 2-amino-2-ethyl-1,3-propanediol, tris(hydroxymethyl)aminomethane and *N*-methylglucamine.

Analogously salts can be formed from compounds of formula I wherein $R^3$ is $—(CH_2)_rC(O)OH$.

The compounds of formula I are also capable of forming non-toxic, pharmaceutically acceptable acid addition salts with inorganic and organic acids. By "non-toxic pharmaceutically acceptable acid addition salts" are meant salts that do not exhibit toxic manifestations at normal therapeutic doses. Examplary of such salts are those formed with such acids as hydrochloric, sulfuric, phosphoric, citric, acetic, propionic, tartaric, maleic, benzoic, cyclopropylcarboxylic, adamantylcarboxylic, lauryl sulfonic, glucoheptonic, stearic and lactobionic acid.

Compounds of the formula I wherein $R^5$ is hydrogen may also exist as tautomers having the formula

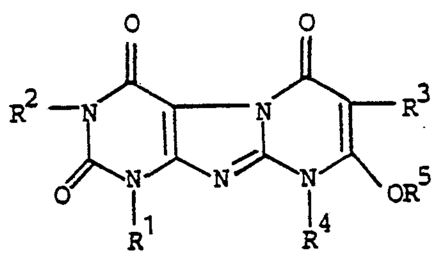

IA

wherein $R^5$ is hydrogen. The term "tautomer of compounds of formula I" covers compounds of formula IA, wherein $R^5$ is hydrogen, acyl containing 2 to 8 carbon atoms or alkyl containing 1 to 4 carbon atoms.

An area of interest are also the compounds of formula I, their tautomers and the pharmaceutically acceptable salts thereof, wherein

$R^1$ and $R^2$ are the same or different and are hydrogen, alkyl having 1 to 8 carbon atoms, cycloalkyl having 3 to 8 carbon atoms, alkenyl having 3 to 8 carbon atoms, aryl;

alkyl (having 1 to 8 carbon atoms) substituted by cycloalkyl (having 3 to 8 carbon atoms), aryl or heterocyclic radical;

$R^3$ is hydrogen, alkyl containing 1 to 8 carbon atoms, acyloxyalkyl (the acyl and alkyl group having 1 to 8 carbon atoms each, the acyloxyalkyl group in toto having 2 to 12 carbon atoms), oxoalkyl containing 1 to 8 carbon atoms, aryl;

alkyl (containing 1 to 8 carbon atoms) substituted by aryl, cyano, halo or heterocyclic radical;

-alkyl—X—$C_nH_{2n+1}$ wherein alkyl contains 1 to 6 carbon atoms, n is an integer from 0 to 4 and X is O,S,$S^+—O^-$, $SO_2$ or —N—$C_pH_{2p+1}$ wherein p is an integer from 0 to 4;

$R^4$ is hydrogen, alkyl containing 1 to 8 carbon atoms, aryl, a heterocyclic radical;

alkyl (containing 1 to 8 carbon atoms) substituted by aryl or a heterocyclic radical;

$R^5$ is hydrogen, acyl containing 2 to 8 carbon atoms or alkyl containing 1 to 4 carbon atoms.

The compounds of this invention can be produced by a variety of processes known in the art:

A) For the preparation of compounds as defined above wherein $R^5$ is hydrogen and $R^1$, $R^2$, $R^3$ and $R^4$ are as defined above:

reaction of an 8-aminoxanthine of formula II

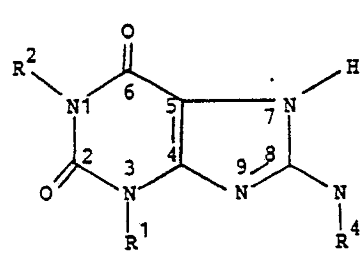

II

with a reactive derivative of $R^3$-substituted malonic acid.

This process can, for example, be carried out by reacting the 8-aminoxanthine (II) with a diester of the $R^3$-containing malonic acid, preferably its diethylester in the presence of a base such as sodium methoxide at elevated temperature (process A1).

This process (A1) is particularly useful for the preparation of compounds wherein $R^3$ is hydrogen, alkyl, cycloalkyl, alkyl substituted by cycloalkyl, and -alkyl-X—$C_nH_{2n+1}$, wherein X is —N—$C_pH_{2p+1}$. The process is not suitable for the preparation wherein $R^3$ is alkenyl, alkynyl or -alkyl-X—$C_nH_{2n+1}$ wherein X is O, S, $S^+$—$O^-$ or $SO_2$.

The 8-aminoxanthine (II) used as starting material in this process can be prepared according to known methods, e.g. as follows:

Step 1:

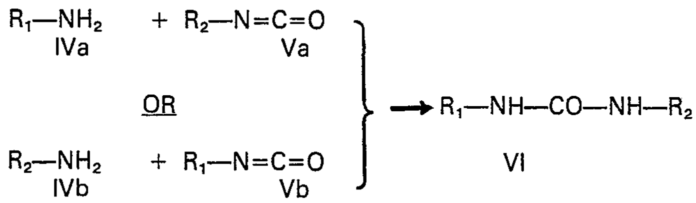

IVa Va OR IVb Vb VI

The ureas of formula VI may be prepared by reacting approximately equimolar quantities of an amine ($R_1$—$NH_2$ or $R_2$—$NH_2$) with an isocyanate ($R_2$—N=C=O or $R_1$—N=C=O) in an inert solvent, e.g., chloroform.

Step 2:

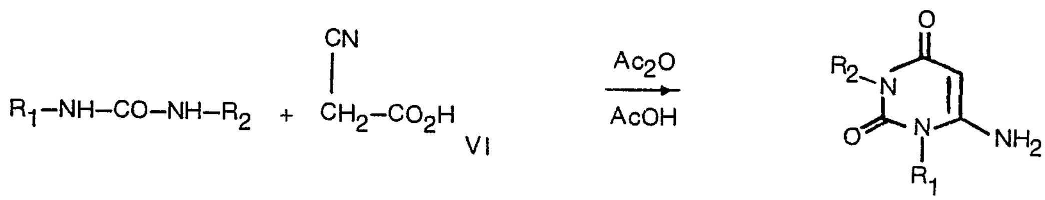


VI VII

Compounds of formula VII may be prepared by the well known Traube purine synthesis or a modification thereof. Equimolar quantities of the compound of formula VI and cyanoacetic acid are heated to 60°C with two equivalents of acetic anhydride using glacial acetic acid as solvent. After 2 to 8 hours as much as possible of the acetic acid and acetic anhydride are removed at 60°C *in vacuo*. The resultant mixture is poured into water and made basic, e.g., with solid sodium carbonate. The mixture is boiled 1—4 hours, then cooled. On standing either a solid will form which may be filtered off and purified, or an oil will form which may be extracted and purified.

For compounds of formula VI where $R_1$ and $R_2$ are different, two differrent compounds of formula VII may be formed, i.e.,

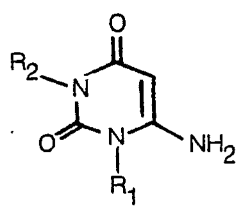

VIIa and

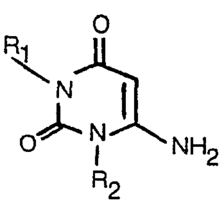

VIIb

These compounds may be separated by fractional crystallization or by chromatography (e.g. column or HPLC).

Step 3:

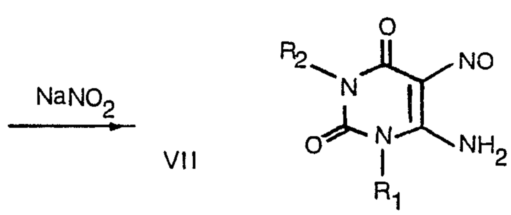


VII VIII

The purified 6-aminouracil compounds of formula VII may be converted to the 5-nitroso-6-aminouracil compounds of formula VIII by combining the 6-aminouracil derivative and sodium nitrite (one equivalent) and boiling in ethanol/water while adding glacial acetic acid. The nitrosocompound of formula VIII which precipitates is then filtered off, washed with water and dried.

Step 4:

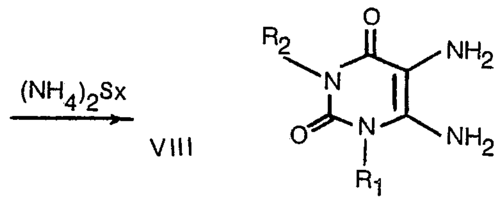


VIII

IX

The 6-amino-5-nitrosouracil compound of formula VIII is reduced to the corresponding 5-amino-compound of formula IX in aqueous suspension by the use of an excess of ammonium polysulfide solution with warming. When the color is discharged, the mixture is cooled and the supernatant liquid is decanted off. The residue is dissolved in methylene chloride, dried and evaporated. The crude product is used in the next step.

Step 5:

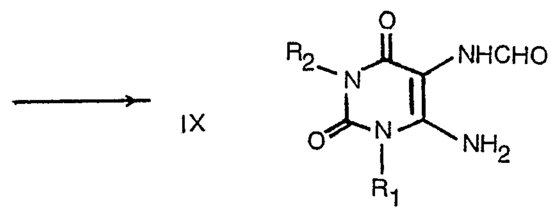

IX

X

The 5,6-diaminouracil compound of formula IX is heated with excess formic acid at 120—150°C for 1—4 hours, then allowed to stand at room temperature overnight. Most of the acid is then removed (75°C; reduced pressure) and the residue is dissolved in hot methanol and filtered. The product of formula X is isolated by chilling and filtering off the resulting solid or by evaporation of the methanol.

Step 6:

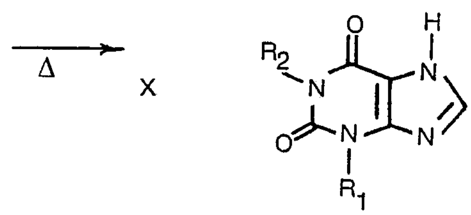


X

XI

The 6-amino-5-formamidouracil compound of formula X is heated to 250—285°C until frothing ceases (10—60 mins.). The product is then cooled and the crude product of formula XI is recrystallized, e.g. from $MeOH/H_2O$.

Step 7:

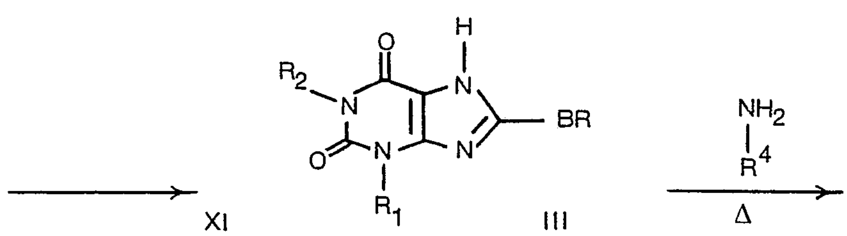


XI

III

II

The xanthine compound of formula XI is dissolved in glacial acetic acid. The solution is warmed gradually to 100°C while a solution of bromine in acetic acid is slowly added until thin layer chromatography shows that starting material has been consumed. The product, a compound of formula III, is isolated by pouring the reaction mixture into water, filtering and recrystallizing, if necessary. The 8-bromoxanthine of formula III is converted to the 8-substituted-amino-xanthine of formula II by heating with excess amine at elevated temperature.

Process A can also be carried out by reacting the 8-aminoxanthine (II) with a half-ester of the $R^3$-containing malonic acid halogenide (preferably chloride) in a suitable solvent (such as for example a mixture of dioxane and acetonitrile) at elevated temperature (process A2). This procedure is particularly

useful for the preparation of compounds wherein $R^3$ is hydrogen. It can not be used for the preparation of compounds wherein $R^3$ is alkenyl, alkynyl or -alkyl-X—$C_nH_{2n+1}$ wherein X is O, S, $S^+$—$O^-$ or $SO_2$.

Process A can also be carried out by reacting the 8-aminoxanthine (II) with a di-ester (preferably diethylester) or the $R^3$-containing malonic acid and a stoichiometric amount of a base (e.g. sodium hydride) in an organic solvent (e.g. dimethyl formamide) (process A3).

B) Compounds of formula I, wherein $R^1$, $R^2$ and $R^4$ are as defined above, $R^5$ is hydrogen and $R^3$ is methyl, ethyl, propyl, alkenyl containing 3 to 8 carbon atoms, cycloalkenyl containing 5 to 8 carbon atoms, alkynyl containing 3 to 8 carbon atoms;

alkyl (containing 1 to 8 carbon atoms) substituted by cyclopropyl, cycloalkenyl (containing 5 to 8 carbon atoms), aryl or cyano; or

—$CH_2$—C(O)$OR^8$ (wherein $R^8$ is alkyl having from 1 to 8 carbon atoms);

whereby (in the groups) alkenyl, cycloalkenyl, alkynyl and substituted alkyl the carbon atom being in α-position to the unsaturation or the —CN group or aryl respectively, is connected with the carbon atom of position 7 of the pyrimido[2,1-f]purine (I) can be prepared by reacting a compound of formula I, wherein $R^1$, $R^2$, $R^4$ and $R^5$ are defined as above and $R^3$ is hydrogen with a compound hal-$R^3$(XIII), wherein hal is halogen, preferably bromo. The reaction can be outlined by the following reaction scheme

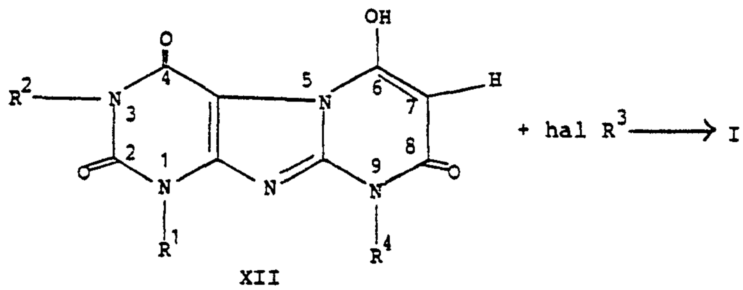

XII

XIII

It is to be noted that in compound XIII wherein $R^3$ is alkenyl, cycloalkenyl, alkynyl or substituted alkyl the halogen is in 2-position to the unsaturation or the —CN group or aryl respectively.

The process is carried out by reacting the compound of formula XII or a sodium salt thereof with the compound hal-$R^3$ (XIII) in the presence of sodium hydride in an aprotic organic solvent such as for example dimethylformamide.

C) Compounds of formula I and their tautomers, wherein

$R^1$ and $R^2$ are the same or different and are hydrogen, alkyl having 1 to 8 carbon atoms, cycloalkyl having 4 to 8 carbon atoms, aryl;

alkyl (having 1 to 8 carbon atoms) substituted by cycloalkyl (having 4 to 8 carbon atoms), aryl or heterocyclic radical;

$R^3$ is alkyl containing 2 to 8 carbon atoms, cycloalkyl containing 5 to 8 carbon atoms;

$R^4$ is hydrogen, alkyl containing 1 to 8 carbon atoms, aryl, a heterocyclic radical;

alkyl (containing 1 to 8 carbon atoms) substituted by cycloalkyl (containing 4 to 8 carbon atoms), aryl or a heterocyclic radical;

$R^5$ is hydrogen, acyl containing 2 to 8 carbon atoms or alkyl containing 1 to 4 carbon atoms;

can be prepared by hydrogenation of corresponding compounds of formula I or their tautomers, wherein $R^1$, $R^2$, $R^4$ and $R^5$ are defined as above and $R^3$ is alkenyl containing 2 to 8 carbon atoms, cycloalkenyl containing 5 to 8 carbon atoms or alkynyl containing 3 to 8 carbon atoms. Preferably the hydrogenation is carried out catalytically. The process can be performed in an aprotic solvent such as dimethylformamide using palladium-on-charcoal, room temperature and 4.05 bar (4 atmospheres) hydrogen pressure.

The compounds obtained by any one of the above described processes can be subjected to one or more of the following after

i) For the preparation of compounds of formula I or their tautomers, wherein $R^5$ is alkyl containing 1 to 4 carbon atoms: alkylation of a compound of formula I or its tautomer, wherein $R^5$ is hydrogen and $R^1$, $R^2$, $R^3$ and $R^4$ are defined as above. The alkylation can be carried out by reacting the compound with diazoalkane (wherein the alkyl group is the desired $R^5$-group).

ii) Esterification of compounds of formula I or their tautomers, wherein $R^3$ is alkyl substituted by hydroxy to form the corresponding acyloxy alkyl-compound.

iii) The compounds of formula I and their tautomers can be subjected to salt formation. The compounds form pharmaceutically acceptable acid addition salts when reacted with acids discussed above. Compounds, wherein $R^5$ is hydrogen and/or $R^3$ is —$(CH_2)_pC(O)OH$ form salts, wherein the hydrogen is replaced by a cation, when reacted with one of the desired bases discussed above.

iv) The compounds of formula I and their tautomers, wherein $R^3$ is —$(CH_2)_rC(O)OR^8$ can be reduced to the corresponding —$(CH_2)_rCH_2OH$ compounds by reduction under standard conditions, using, for example, lithiumborohydride.

The following examples further illustrate the preparation of the compounds of this invention.

## Example 1

9-Benzyl-1,3-dimethyl-6-hydroxy-7-n-propyl-9H-8-oxo-pyrimido[2,1-f]purine-2,4-dione

Heat together a mixture of one equivalent of 8-bromotheophylline with three to four equivalents of benzylamine at 160—180°C until thin layer chromatography analysis shows that no starting compound remains. Cool. Triturate with ethanol and water to yield 8-benzylamino-theophylline. (If desired, the reaction is carried out in a sealed vessel).

Suspend 8-benzylamino-theophylline (10 g) in diethyl n-propyl malonate (65 ml). Add sodium methoxide (0.7 g), and stir and heat to about 200°C (bath temperature). Separate the ethanol which is formed with a Dean and Stark trap. After about 4 to 6 hours, raise the bath temperature to about 215°C until no more starting material is present (as shown by thin layer chromatography).

Cool to below 60°C and add ethanol. Stir and triturate and then filter, wash and air dry. Recrystallize the product from acetonitrile (about 60 parts). Wash with ether and dry *in vacuo* at 70 to 75°C to yield the title compound having a melting point of 217°C.

## Example 2

7-(2-Acetoxyethyl)-9-benzyl-1,3-dimethyl-6-hydroxy-9H-8-oxo-pyrimido[2,1-f]purine-2,4-dione

Suspend 9-benzyl-1,3-dimethyl-7-(2-hydroxyethyl)-6-hydroxy-9*H*-8-oxo-pyrimido[2,1-f]purine-2,4-dione (1 g) in pyridine (10 ml) at 0°C. Add acetic anhydride (3 ml). Stir at 0—5°C overnight. Pour onto ice water, filter, wash and recrystallize the product ($MeOH/H_2O$) to yield the title compound.

## Example 3

9-(4-Fluorobenzyl)-1,3,7-trimethyl-6-acetoxy-9H-8-oxopyrimido[2,1-f]purine 2,4-dione

Dissolve 9-(4-fluorobenzyl)-1,3,7-trimethyl-6-hydroxy-9*H*-8-oxopyrimido[2,1-f]purine-2,4-dione (1 g) in pyridine (20 ml) containing acetic anhydride (8 ml). Heat the solution to reflux overnight, then pour into 10% HCl solution. Wash the solids, dry, and chromatograph on silica gel in methylene chloride (95%):acetone (5%) to yield the title compound, m.p. 230.5°C, as the hemihydrate.

In a similar manner, prepare any of the compounds of this invention wherein $R^5$ is acyl including aroyl.

## Example 4

1,3-Dimethyl-6-methoxy-9-phenylmethyl-7-n-propylpyrimido[2,1-f]purine-2,4,8-(1H,3H,9H)trione

1,3-Dimethyl-6-hydroxy-9-phenylmethyl-7-n-propylpyrimido[2,1-f]purine-2,4,8-(1H,3H,9H)trione (3 g) dissolved in 200 ml chloroform at 0°C is treated with an ethereal solution of diazomethane. The solution is stirred at 0°C for 1.5 hr and the excess diazomethane is destroyed by the addition of acetic acid. The chloroform solution is washed with a solution of sodium bicarbonate and the chloroform is removed under reduced pressure. The solid obtained is chromatographed on silica gel using 1% methanol in chloroform to give the title compound, m.p. 199—201°C.

## Example 5

9-Benzyl-1,3-dimethyl-7-(β-ethoxyethyl)-6-hydroxypryrimido[2,1-f]purine-2,4,8-(1H,3H,9H)trione

To a stirred suspension of 7.43 g (26.0 mmoles) of 8-benzylaminotheophylline in 104 ml of dry N,N-dimethylformamide is added portionwise over 10 minutes 1.19 g (29.0 mmoles) of a 60% dispersion of sodium hydride. The mixture is heated at 50°C under nitrogen for 30 minutes before adding 13.3 g (57.0 mmoles) of the diethyl ester of β-ethoxyethyl malonic acid. The resultant mixture is heated at 150°C under nitrogen for approximately 37 hours and is then allowed to cool. Solvent is removed *in vacuo,* and the residual semisolid is treated with 250 ml of chloroform and 100 ml of water and is acidified with 3M hydrochloric acid. The organic layer is separated, and the aqueous layer is extracted with 2 × 100 ml of chloroform. The combined chloroform extracts are washed with 3 × 100 ml of water and 125 ml of brine, dried over anhydrous sodium sulfate, and stripped of solvent under vacuum. The residue is triturated with ether and filtered. The crude product thus obtained is chromatographed on silica gel, eluting first with chloroform and then with chloroform containing 5% ammonia. Thus is obtained the title compound with m.p. 156.5—157.5°C.

## Example 6

9-Benzyl-1,3-dimethyl-6-hydroxypyrimido[2,1-f]purine-2,4,8-(1H,3H,9H)trione

A stirred mixture of 30 g (0.105 mole) of 8-benzylaminotheophylline and 35.1 g (0.232 mole) of ethyl malonyl chloride in 600 ml of 1:1 dioxane-acetonitrile is refluxed under nitrogen until the benzylamino-theophylline has been consumed (ca. 3.5 hr).

The reaction mixture is cooled to R.T. and poured into 800 ml of ether. The resultant precipitate is filtered and washed with ether to obtain the title compound with m.p. 205.5—209°C.

Example 7

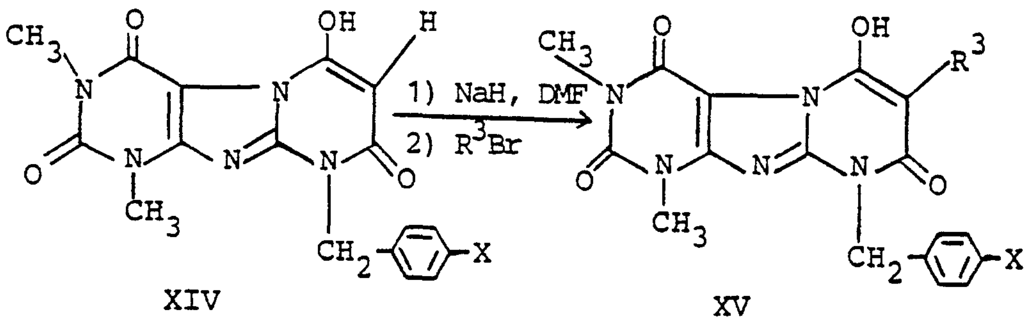


XIV XV

The sodium salt of (XIV) is generally formed *in situ*, as indicated above; alternatively, the preformed sodium salt of (XIV) may be used.

To compound (XIV) in N,N-dimethylformamide (solution or suspension) is added an equivalent of sodium hydride, and the mixture is stirred under a nitrogen atmosphere at R.T. for 15—30 minutes. A slight excess of the $R^3Br$ is then added, and the mixture is allowed to stir under a nitrogen atmosphere at the temperature indicated in the TABLE.

At the end of the reaction period, the mixture is worked up according to either of the two following methods:

METHOD A:

Pour the reaction mixture into ice-water. Collect the moist solid that precipitates, and dissolve it in chloroform. Wash the chloroform solution with water, dry the solution ($MgSO_4$ or $Na_2SO_4$), strip off solvent *in vacuo*, and chromatograph on silica gel, then recrystallize.

METHOD B:

Strip the DMF *in vacuo* from the reaction mixture. Triturate the residue in ether. Filter the solids, dissolve them in chloroform-methanol-ammonia and chromatograph on silica gel, and recrystallize.

The so obtained compounds can be transferred into their sodium salts:

Stir for approximately two hours at room temperature the purified free acid form of the compound in an aqueous solution of slightly less than one equivalent of sodium hydroxide. Filter the mixture and lyophilize the filtrate to obtain the sodium salt.

Compounds of formula XV are prepared according to this example as shown in the following Table I (physical data of the compounds are given in Table II):

| Compound | X | $R^3$ | Solvent | Reaction Temp. °C |
|---|---|---|---|---|
| 22 | H | $-CH_2CH=CH_2$ | DMF | Room Temperature |
| 23 | H | $-CH_2C\equiv CH$ | DMF 18-Crown-6 | Room Temperature |
| 25 | H | $-CH_2CH=CHCH_3$ | DMF | Room Temperature |
| 30 | H | 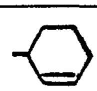 | DMF | Room Temperature |
| 24 | H | $-CH_2CH=C(CH_3)_2$ | DMF | Room Temperature |
| 26 | H | $-CH_2-$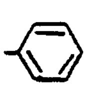 | DMF 18-Crown-6 | Room Temperature |
| 32 | H | $-CH_2CO_2CH_3$ | DMF 18-Crown-6 | 80 |
| 31 | H | $-CH_2CN$ | DMF | 80 |
| 69 | F | $-CH_2CH=C(CH_2)_2$ | DMF | Room Temperature |

The following table (Table II) summarizes examples of compounds of formula I

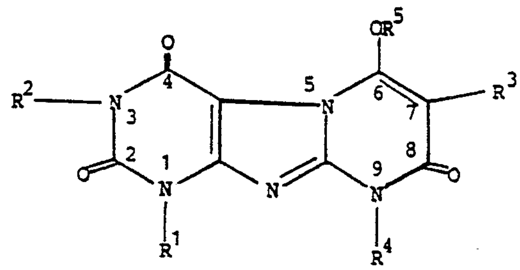

(I)

or its tautomers. The compound can be prepared according to the processes and examples disclosed. In the table "Ph" stands for phenyl.

| No. | $R_1$ | $R_2$ | $R_3$ | $R_4$ | $R_5$ | m.p. |
|---|---|---|---|---|---|---|
| 1 | $-CH_3$ | $-CH_3$ | $-CH_2CH_2CH_3$ | $-CH_2-Ph$ | H | 217°C |
| 2 | $-CH_3$ | $-CH_3$ | $-CH_3$ | $-CH_2-Ph$ | H | 204°C |
| 3 | $-CH_3$ | $-CH_3$ | $-(CH_2)_3CH_3$ | $-CH_2-Ph$ | H | 162°C |
| 4 | $-CH_3$ | $-CH_3$ | $-CH_2CH(CH_3)_2$ | $-CH_2-Ph$ | H | 168°C |
| 5 | $-CH_3$ | $-CH_3$ | $-CH_3$ | $-Ph$ | H | >260°C |
| 6 | $-CH_3$ | $-CH_3$ | $-CH_2CH_2CH_3$ | $-Ph$ | H | >260°C |
| 7 | $-CH_3$ | $-CH_3$ | $-CH_2(CH_2)_2CH_3$ | $-Ph$ | H | 227°C |
| 8 | $-CH_3$ | $-CH_3$ | $-CH_2CH(CH_3)_2$ | $-Ph$ | H | >260°C |
| 9 | $-CH_3$ | $-CH_3$ | $-CH_2(CH_2)_3CH_3$ | $-Ph$ | H | 219°C |
| 10 | $-CH_3$ | $-CH_3$ | $-CH_3$ | 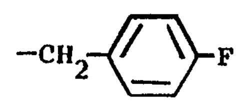 | H | 248°C sinters |

| No. | $R_1$ | $R_2$ | $R_3$ | $R_4$ | $R_5$ | m.p. |
|---|---|---|---|---|---|---|
| 11 | $-CH_3$ | $-CH_3$ | $-CH_2CH_2CH_3$ | 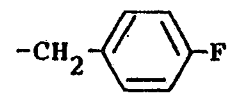 | H | 215°C |
| 12 | $-CH_3$ | $-CH_3$ | $-CH_2CH_2CH_3$ | 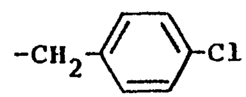 | H | 195°C |
| 13 | $-CH_3$ | $-CH_3$ | $-CH_2CH_2CH_3$ | 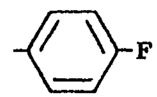 | H | >260°C |
| 14 | $-CH_3$ | $-CH_3$ | $-CH_2(CH_2)_2CH_3$ | 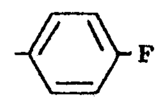 | H | >260°C |
| 15 | $-CH_3$ | $-CH_3$ | $-CH_2CH_2CH_3$ | 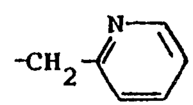 | H | 208°C |

| No. | $R_1$ | $R_2$ | $R_3$ | $R_4$ | $R_5$ | m.p. |
|---|---|---|---|---|---|---|
| 16 | $—CH_3$ | $—CH_3$ | $—CH_2—Ph$ | 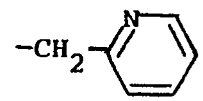 | H | 199°C |
| 17 | $—(CH_2)_3CH_3$ | $—(CH_2)_3CH_3$ | $—CH_3$ | $—CH_2—Ph$ | H | 138°C |
| 18 | $—CH_3$ | $—CH_3$ | —H | $—CH_2—Ph$ | H | 209—222°C |
| 19 | $—CH_3$ | $—CH_3$ | $—CH_2CH_3$ | $—CH_2—Ph$ | H | |
| 20 | $—CH_3$ | $—CH_3$ | $—CH_2(CH_2)_3CH_3$ | $—CH_2—Ph$ | H | |
| 21 | $—CH_3$ | $—CH_3$ | $—CH_2CH_2CH(CH_3)_2$ | $—CH_2—Ph$ | H | |
| 22 | $—CH_3$ | $—CH_3$ | $—CH_2(CH_2)_4CH_3$ | $—CH_2—Ph$ | H | |
| 23 | $—CH_3$ | $—CH_3$ | $—CH_2(CH_2)_5CH_3$ | $—CH_2—Ph$ | H | |
| 24 | $—CH_3$ | $—CH_3$ | $—CH_2(CH_2)_6CH_3$ | $—CH_2—Ph$ | H | |
| 25 | $—CH_3$ | $—CH_3$ | —Ph | $—CH_2—Ph$ | H | |

| No. | $R_1$ | $R_2$ | $R_3$ | $R_4$ | $R_5$ | m.p. |
|---|---|---|---|---|---|---|
| 26 | $-CH_3$ | $-CH_3$ | $-CH_2-Ph$ | $-CH_2-Ph$ | H | |
| 27 | $-CH_3$ | $-CH_3$ | $-CH_2CH_2-Ph$ | $-CH_2-Ph$ | H | |
| 28 | $-CH_3$ | $-CH_3$ | $-CH(CH_3)_2$ | $-CH_2-Ph$ | H | 167—168.5°C |
| 29 | $-CH_3$ | $-CH_3$ | $-(CH_3)CHC_2H_5$ | $-CH_2-Ph$ | H | |
| 30 | $-CH_3$ | $-CH_3$ | $-CH_2CH_3$ | $-Ph$ | H | |
| 31 | $-CH_3$ | $-CH_3$ | $-CH_2(CH_2)_4CH_3$ | $-Ph$ | H | |
| 32 | $-CH_3$ | $-CH_3$ | $-CH_2(CH_2)_5CH_3$ | $-Ph$ | H | |
| 33 | $-CH_3$ | $-CH_3$ | $-CH_2(CH_2)_6CH_3$ | $-Ph$ | H | |
| 34 | $-CH_3$ | $-CH_3$ | $-CH_2CH_3$ | 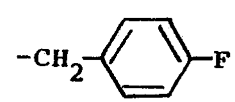 | H | |
| 35 | $-CH_3$ | $-CH_3$ | $-CH(CH_3)_2$ | 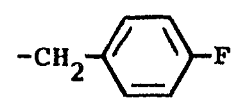 | H | |

| No. | $R_1$ | $R_2$ | $R_3$ | $R_4$ | $R_5$ | m.p. |
|---|---|---|---|---|---|---|
| 36 | $—CH_3$ | $—CH_3$ | $—CH_2(CH_2)_2CH_3$ | 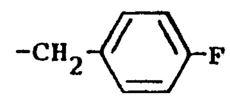 | H | 184.5—186.5°C |
| 37 | $—CH_3$ | $—CH_3$ | $—CH_2CH(CH_3)_2$ | 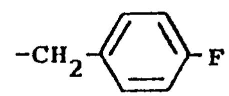 | H | |
| 38 | $—CH_3$ | $—CH_3$ | $—(CH_3)CHC_2H_5$ | 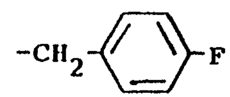 | H | |
| 39 | $—CH_3$ | $—CH_3$ | $—CH_2(CH_2)_3CH_3$ | 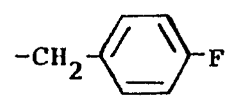 | H | |
| 40 | $—CH_3$ | $—CH_3$ | $—CH_2CH_2CH(CH_3)_2$ | 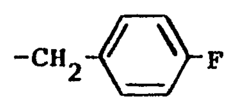 | H | |

| No. | $R_1$ | $R_2$ | $R_3$ | $R_4$ | $R_5$ | m.p. |
|---|---|---|---|---|---|---|
| 41 | $-CH_3$ | $-CH_3$ | $-CH_2(CH_2)_4CH_3$ | 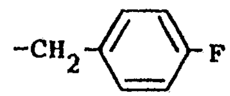 | H | |
| 42 | $-CH_3$ | $-CH_3$ | —Ph | 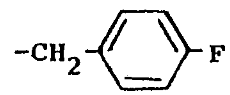 | H | 228—229°C |
| 43 | $-CH_3$ | $-CH_3$ | $-CH_2$—Ph | 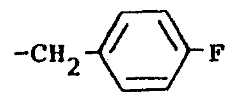 | H | 247—248.5°C |
| 44 | $-CH_3$ | $-CH_3$ | $-CH_2CH_2$—Ph | 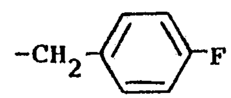 | H | |
| 45 | $-CH_3$ | $-CH_3$ | $-CH_3$ | 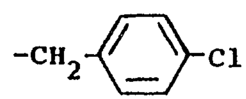 | H | |

| No. | $R_1$ | $R_2$ | $R_3$ | $R_4$ | $R_5$ | m.p. |
|---|---|---|---|---|---|---|
| 46 | $-CH_3$ | $-CH_3$ | $-CH_2CH_3$ | 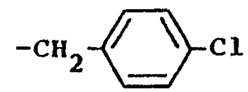 | H | |
| 47 | $-CH_3$ | $-CH_3$ | $-CH(CH_3)_2$ | 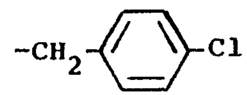 | H | |
| 48 | $-CH_3$ | $-CH_3$ | $-CH_2(CH_2)_2CH_3$ | 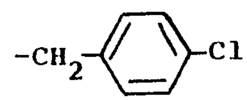 | H | 178—180.5°C |
| 49 | $-CH_3$ | $-CH_3$ | $-CH_2CH(CH_3)_2$ | 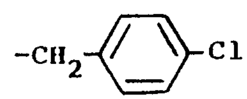 | H | |
| 50 | $-CH_3$ | $-CH_3$ | $(CH_3)CHC_2H_5$ | 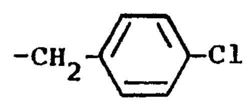 | H | |

| No. | $R_1$ | $R_2$ | $R_3$ | $R_4$ | $R_5$ | m.p. |
|---|---|---|---|---|---|---|
| 51 | $-CH_3$ | $-CH_3$ | $-CH_2(CH_2)_3CH_3$ | 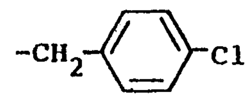 | H | |
| 52 | $-CH_3$ | $-CH_3$ | $-CH_2CH_2CH(CH_3)_2$ | 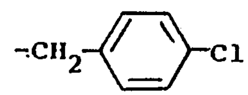 | H | |
| 53 | $-CH_3$ | $-CH_3$ | $-CH_2(CH_2)_4CH_3$ | 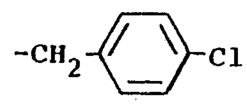 | H | |
| 54 | $-CH_3$ | $-CH_3$ | $-CH_3$ | 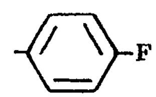 | H | |
| 55 | $-CH_3$ | $-CH_3$ | $-CH_2CH_3$ | 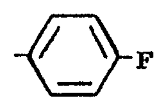 | H | |

| No. | $R_1$ | $R_2$ | $R_3$ | $R_4$ | $R_5$ | m.p. |
|---|---|---|---|---|---|---|
| 56 | $-CH_3$ | $-CH_3$ | $-CH(CH_3)_2$ | 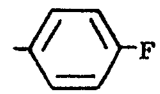 | H | |
| 57 | $-CH_3$ | $-CH_3$ | $-CH_2CH(CH_3)_2$ | 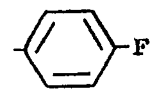 | H | |
| 58 | $-CH_3$ | $-CH_3$ | $-CH_2(CH_2)_3CH_3$ | 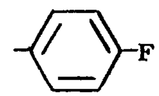 | H | |
| 59 | $-CH_3$ | $-CH_3$ | $-CH_2(CH_2)_4CH_3$ | 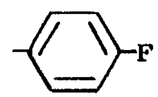 | H | |
| 60 | $-CH_3$ | $-CH_3$ | —Ph | 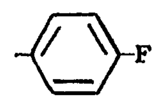 | H | |

| No. | $R_1$ | $R_2$ | $R_3$ | $R_4$ | $R_5$ | m.p. |
|---|---|---|---|---|---|---|
| 61 | $-CH_3$ | $-CH_3$ | $-CH_2-Ph$ | 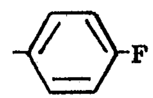 | H | |
| 62 | $-CH_3$ | $-CH_3$ | $-CH_3$ | 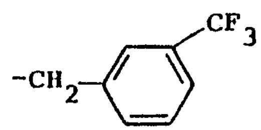 | H | |
| 63 | $-CH_3$ | $-CH_3$ | $-CH_2CH_2CH_3$ | 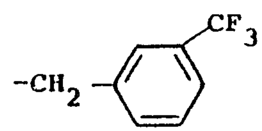 | H | |
| 64 | $-CH_3$ | $-CH_3$ | $-CH_2(CH_2)_2CH_3$ | 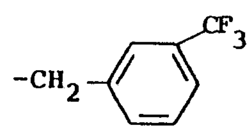 | H | |
| 65 | $-CH_3$ | $-CH_3$ | $-CH_2CH(CH_3)_2$ | 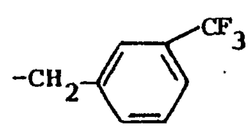 | H | |

| No. | $R_1$ | $R_2$ | $R_3$ | $R_4$ | $R_5$ | m.p. |
|---|---|---|---|---|---|---|
| 66 | $-CH_3$ | $-CH_3$ | $-CH_2(CH_2)_3CH_3$ | 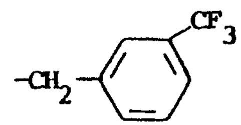 | H | |
| 67 | $-CH_3$ | $-CH_3$ | $-CH_3$ | 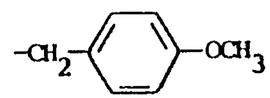 | H | |
| 68 | $-CH_3$ | $-CH_3$ | $-CH_2CH_2CH_3$ | 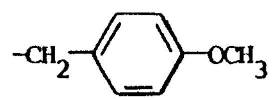 | H | 207—208°C |
| 69 | $-CH_3$ | $-CH_3$ | $-CH_2(CH_2)_2CH_3$ | 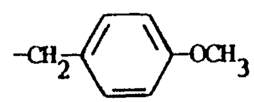 | H | |
| 70 | $-CH_3$ | $-CH_3$ | $-CH_2(CH_2)_3CH_3$ | 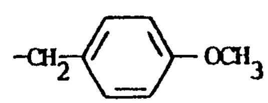 | H | |

| No. | $R_1$ | $R_2$ | $R_3$ | $R_4$ | $R_5$ | m.p. |
|---|---|---|---|---|---|---|
| 71 | $-CH_3$ | $-CH_3$ | $-CH_3$ | 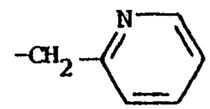 | H | |
| 72 | $-CH_3$ | $-CH_3$ | $-CH_2(CH_2)_2CH_3$ | 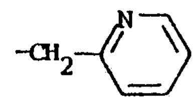 | H | |
| 73 | $-CH_3$ | $-CH_3$ | $-CH_2(CH_2)_3CH_3$ | 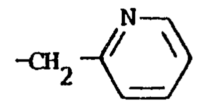 | H | |
| 74 | $-CH_3$ | $-CH_3$ | $-Ph$ | 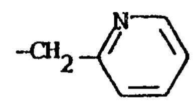 | H | |
| 75 | $-CH_3$ | $-CH_3$ | $-CH_3$ | $-CH_2(CH_2)_2CH_3$ | H | |
| 76 | $-CH_3$ | $-CH_3$ | $-CH_2CH_2CH_3$ | $-CH_2(CH_2)_2CH_3$ | H | |
| 77 | $-CH_3$ | $-CH_3$ | $-CH_2CH(CH_3)_2$ | $-CH_2(CH_2)_2CH_3$ | H | |
| 78 | $-CH_3$ | $-CH_3$ | $-Ph$ | $-CH_2(CH_2)_2CH_3$ | H | |
| 79 | $-CH_3$ | $-CH_3$ | $-CH_2-Ph$ | $-CH_2(CH_2)_2CH_3$ | H | |
| 80 | $-CH_3$ | $-CH_3$ | 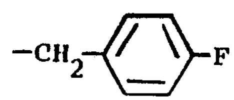 | $-CH_2(CH_2)_2CH_3$ | H | |

| No. | $R_1$ | $R_2$ | $R_3$ | $R_4$ | $R_5$ | m.p. |
|---|---|---|---|---|---|---|
| 81 | $-CH_3$ | $-CH_3$ | $-CH_3$ | 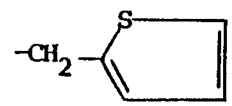 | H | |
| 82 | $-CH_3$ | $-CH_3$ | $-CH_2CH_3$ | 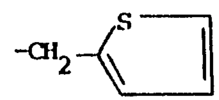 | H | |
| 83 | $-CH_3$ | $-CH_3$ | $-CH_2CH_2CH_3$ | 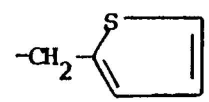 | H | 225—226°C |
| 84 | $-CH_3$ | $-CH_3$ | $-CH_2(CH_2)_2CH_3$ | 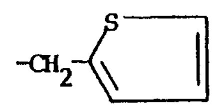 | H | |
| 85 | $-CH_3$ | $-CH_3$ | $-CH_2CH(CH_3)_2$ | 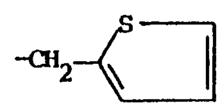 | H | |

| No. | $R_1$ | $R_2$ | $R_3$ | $R_4$ | $R_5$ | m.p. |
|---|---|---|---|---|---|---|
| 86 | $-CH_3$ | $-CH_3$ | $-CH_2(CH_2)_3CH_3$ | 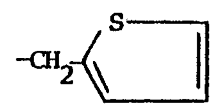 | H | |
| 87 | $-CH_3$ | $-CH_3$ | $-CH_2(CH_2)_4CH_3$ | 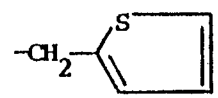 | H | |
| 88 | $-CH_3$ | $-CH_3$ | $-Ph$ | 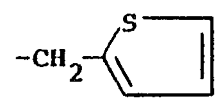 | H | |
| 89 | $-CH_3$ | $-CH_3$ | $-CH_2-Ph$ | 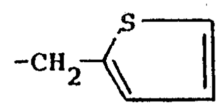 | H | |
| 90 | $-CH_3$ | $-CH_3$ | $-CH_3$ | 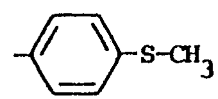 | H | |

| No. | $R_1$ | $R_2$ | $R_3$ | $R_4$ | $R_5$ | m.p. |
|---|---|---|---|---|---|---|
| 91 | $-CH_3$ | $-CH_3$ | $-CH_2CH_2CH_3$ | 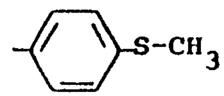 | H | |
| 92 | $-CH_3$ | $-CH_3$ | $-CH_2(CH_2)_2CH_3$ | 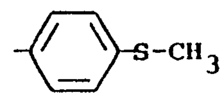 | H | |
| 93 | $-CH_3$ | $-CH_3$ | $-CH_2CH(CH_3)_2$ | 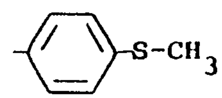 | H | |
| 94 | $-CH_3$ | $-CH_3$ | $-CH_2(CH_2)_3CH_3$ | 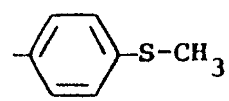 | H | |
| 95 | $-CH_3$ | $-CH_3$ | —Ph | 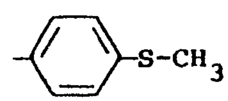 | H | |

| No. | $R_1$ | $R_2$ | $R_3$ | $R_4$ | $R_5$ | m.p. |
|---|---|---|---|---|---|---|
| 96 | $-CH_3$ | $-CH_3$ | $-CH_2-Ph$ | 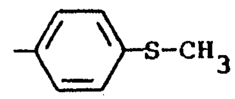 | H | |
| 97 | $-CH_2CH_3$ | $-CH_2CH_3$ | $-CH_3$ | $-CH_2-Ph$ | H | |
| 98 | $-CH_2CH_3$ | $-CH_2CH_3$ | $-CH_2CH_2CH_3$ | $-CH_2-Ph$ | H | |
| 99 | $-CH_2CH_3$ | $-CH_2CH_3$ | $-CH_2(CH_2)_2CH_3$ | $-CH_2-Ph$ | H | |
| 100 | $-CH_2CH_3$ | $-CH_2CH_3$ | $-CH_2CH(CH_3)_2$ | $-CH_2-Ph$ | H | |
| 101 | $-CH_2CH_3$ | $-CH_2CH_3$ | $-CH_2(CH_2)_3CH_3$ | $-CH_2-Ph$ | H | |
| 102 | $-CH_2CH_3$ | $-CH_2CH_3$ | $-CH_2CH_2CH(CH_3)_2$ | $-CH_2-Ph$ | H | |
| 103 | $-CH_2CH_3$ | $-CH_2CH_3$ | $-CH_2(CH_2)_4CH_3$ | $-CH_2-Ph$ | H | |
| 104 | $-CH_2CH_3$ | $-CH_2CH_3$ | $-Ph$ | $-CH_2-Ph$ | H | |
| 105 | $-CH_2CH_3$ | $-CH_2CH_3$ | $-CH_3$ | $-Ph$ | H | |
| 106 | $-CH_2CH_3$ | $-CH_2CH_3$ | $-CH_2CH_2CH_3$ | $-Ph$ | H | |
| 107 | $-CH_2CH_3$ | $-CH_2CH_3$ | $-CH(CH_3)_2$ | $-Ph$ | H | |
| 108 | $-CH_2CH_3$ | $-CH_2CH_3$ | $-CH_2(CH_2)_2CH_3$ | $-Ph$ | H | |
| 109 | $-CH_2CH_3$ | $-CH_2CH_3$ | $-CH_2CH(CH_3)_2$ | $-Ph$ | H | |
| 110 | $-CH_2CH_3$ | $-CH_2CH_3$ | $-CH_2(CH_2)_3CH_3$ | $-Ph$ | H | |

| No. | $R_1$ | $R_2$ | $R_3$ | $R_4$ | $R_5$ | m.p. |
|---|---|---|---|---|---|---|
| 111 | $-CH_2CH_3$ | $-CH_2CH_3$ | $-CH_2CH_2CH(CH_3)_2$ | —Ph | H | |
| 112 | $-CH_2CH_3$ | $-CH_2CH_3$ | $-CH_2(CH_2)_4CH_3$ | —Ph | H | |
| 113 | $-CH_2CH_3$ | $-CH_2CH_3$ | $-CH_3$ | 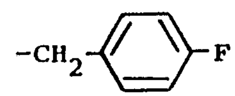 | H | |
| 114 | $-CH_2CH_3$ | $-CH_2CH_3$ | $-CH_2CH_2CH_3$ | 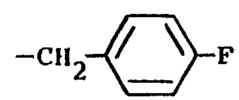 | H | |
| 115 | $-CH_2CH_3$ | $-CH_2CH_3$ | $-CH_2(CH_2)_2CH_3$ | 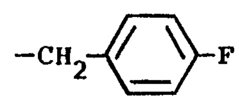 | H | |

| No. | $R_1$ | $R_2$ | $R_3$ | $R_4$ | $R_5$ | m.p. |
|---|---|---|---|---|---|---|
| 116 | $-CH_2CH_3$ | $-CH_2CH_3$ | $-CH_2CH(CH_3)_2$ | 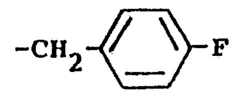 | H | |
| 117 | $-CH_2CH_3$ | $-CH_2CH_3$ | $-CH_2(CH_2)_3CH_3$ | 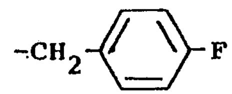 | H | |
| 118 | $-CH_2CH_3$ | $-CH_2CH_3$ | $-CH_3$ | 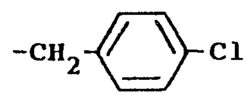 | H | |
| 119 | $-CH_2CH_3$ | $-CH_2CH_3$ | H | 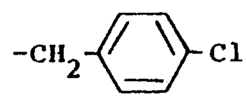 | H | |
| 120 | $-CH_2CH_3$ | $-CH_2CH_3$ | $-CH_2CH_3$ | 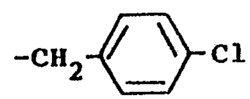 | H | |

| No. | $R_1$ | $R_2$ | $R_3$ | $R_4$ | $R_5$ | m.p. |
|---|---|---|---|---|---|---|
| 121 | $-CH_2CH_3$ | $-CH_2CH_3$ | $-CH_2CH_2CH_3$ | 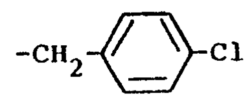 | H | |
| 122 | $-CH_2CH_3$ | $-CH_2CH_3$ | $-CH_2(CH_2)_2CH_3$ | 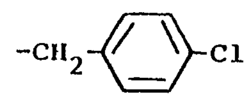 | H | |
| 123 | $-CH_2CH_3$ | $-CH_2CH_3$ | $-CH_3$ | 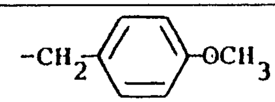 | H | |
| 124 | $-CH_2CH_3$ | $-CH_2CH_3$ | $-CH_2CH_3$ | 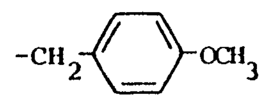 | H | |
| 125 | $-CH_2CH_3$ | $-CH_2CH_3$ | $-CH_2CH_2CH_3$ | 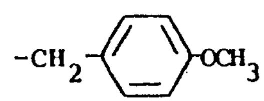 | H | |

| No. | $R_1$ | $R_2$ | $R_3$ | $R_4$ | $R_5$ | m.p. |
|---|---|---|---|---|---|---|
| 126 | $-CH_2CH_3$ | $-CH_2CH_3$ | $-CH_2(CH_2)_2CH_3$ | 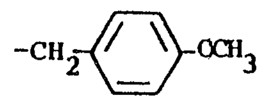 | H | |
| 127 | $-CH_2CH_3$ | $-CH_2CH_3$ | $-CH_2(CH_2)_4CH_3$ | 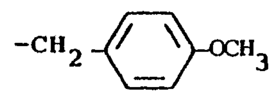 | H | |
| 128 | $-CH_2CH_3$ | $-CH_2CH_3$ | —Ph | $-CH_2(CH_2)_2CH_3$ | H | |
| 129 | $-CH_2CH_3$ | $-CH_2CH_3$ | $-CH_2-$Ph | $-CH_2(CH_2)_2CH_3$ | H | |
| 130 | $-CH_2CH_3$ | $-CH_2CH_3$ | $-CH_2-CH_2-$Ph | $-CH_2(CH_2)_2CH_3$ | H | |

| No. | $R_1$ | $R_2$ | $R_3$ | $R_4$ | $R_5$ | m.p. |
|---|---|---|---|---|---|---|
| 131 | $-CH_2CH_3$ | $-CH_2CH_3$ | 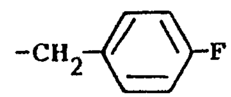 | $-CH_2(CH_2)_2CH_3$ | H | |
| 132 | $-CH_2CH_3$ | $-CH_2CH_3$ | 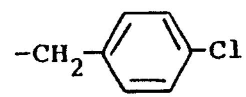 | $-CH_2(CH_2)_2CH_3$ | H | |
| 133 | $-CH_2CH_3$ | $-CH_2CH_3$ | 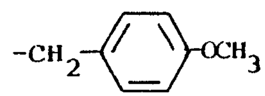 | $-CH_2(CH_2)_2CH_3$ | H | |
| 134 | $-CH_2CH_3$ | $-CH_2CH_3$ | $-CH_3$ | 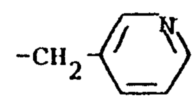 | H | |
| 135 | $-CH_2CH_3$ | $-CH_2CH_3$ | $-CH_2CH_2CH_3$ | 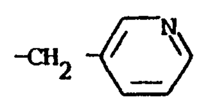 | H | |

| No. | $R_1$ | $R_2$ | $R_3$ | $R_4$ | $R_5$ | m.p. |
|---|---|---|---|---|---|---|
| 136 | $-CH_2CH_3$ | $-CH_2CH_3$ | $-CH_2(CH_2)_2CH_3$ | 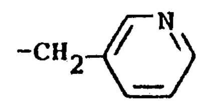 | H | |
| 137 | $-CH_2CH_3$ | $-CH_2CH_3$ | $-CH_2CH(CH_3)_2$ | 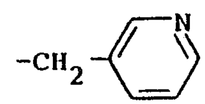 | H | |
| 138 | $-CH_2CH_3$ | $-CH_2CH_3$ | $-CH_2(CH_2)_3CH_3$ | 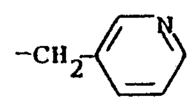 | H | |
| 139 | $-CH_2CH_2CH_3$ | $-CH_2CH_2CH_3$ | $-CH_3$ | $-CH_2-Ph$ | H | |
| 140 | $-CH_2CH_2CH_3$ | $-CH_2CH_2CH_3$ | $-CH_2CH_3$ | $-CH_2-Ph$ | H | |

| No. | $R_1$ | $R_2$ | $R_3$ | $R_4$ | $R_5$ | m.p. |
|---|---|---|---|---|---|---|
| 141 | $-CH_2CH_2CH_3$ | $-CH_2CH_2CH_3$ | $-CH_2CH_2CH_3$ | $-CH_2-Ph$ | H | |
| 142 | $-CH_2CH_2CH_3$ | $-CH_2CH_2CH_3$ | $-CH_2(CH_2)_2CH_3$ | $-CH_2-Ph$ | H | |
| 143 | $-CH_2CH_2CH_3$ | $-CH_2CH_2CH_3$ | $-Ph$ | $-CH_2-Ph$ | H | |
| 144 | $-CH_2CH_2CH_3$ | $-CH_2CH_2CH_3$ | $-CH_3$ | 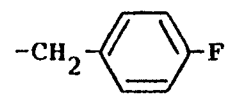 | H | |
| 145 | $-CH_2CH_2CH_3$ | $-CH_2CH_2CH_3$ | $-CH_2CH_3$ | 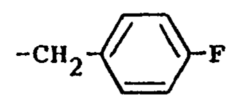 | H | |

| No. | $R_1$ | $R_2$ | $R_3$ | $R_4$ | $R_5$ | m.p. |
|---|---|---|---|---|---|---|
| 146 | $-CH_2CH_2CH_3$ | $-CH_2CH_2CH_3$ | $-CH_2CH_2CH_3$ | 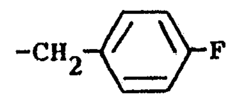 | H | |
| 147 | $-CH_2CH_2CH_3$ | $-CH_2CH_2CH_3$ | $-CH_2(CH_2)_2CH_3$ | 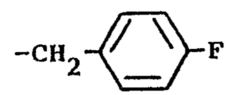 | H | |
| 148 | $-CH_2CH_2CH_3$ | $-CH_2CH_2CH_3$ | $-CH_2CH(CH_3)_2$ | 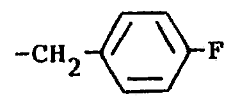 | H | |
| 149 | $-CH_2CH_2CH_3$ | $-CH_2CH_2CH_3$ | —Ph | 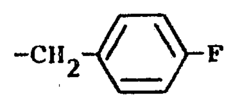 | H | |
| 150 | $-CH_2CH_2CH_3$ | $-CH_2CH_2CH_3$ | $-CH_2$—Ph | 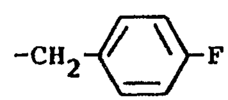 | H | |

| No. | $R_1$ | $R_2$ | $R_3$ | $R_4$ | $R_5$ | m.p. |
|---|---|---|---|---|---|---|
| 151 | $-CH_2CH_2CH_3$ | $-CH_2CH_2CH_3$ | $-CH_3$ | 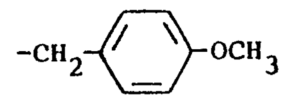 | H | |
| 152 | $-CH_2CH_2CH_3$ | $-CH_2CH_2CH_3$ | $-CH_2CH_3$ | 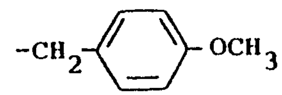 | H | |
| 153. | $-CH_2CH_2CH_3$ | $-CH_2CH_2CH_3$ | $-CH_2CH_2CH_3$ | 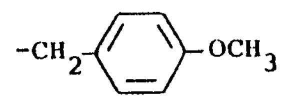 | H | |
| 154 | $-CH_2CH_2CH_3$ | $-CH_2CH_2CH_3$ | $-CH_2(CH_2)_2CH_3$ | 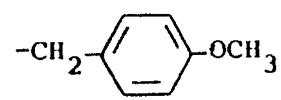 | H | |
| 155 | $-CH_2CH_2CH_3$ | $-CH_2CH_2CH_3$ | $-CH_2CH(CH_3)_2$ | 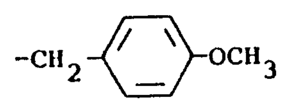 | H | |

| No. | $R_1$ | $R_2$ | $R_3$ | $R_4$ | $R_5$ | m.p. |
|---|---|---|---|---|---|---|
| 156 | $-CH_2CH_2CH_3$ | $-CH_2CH_2CH_3$ | $-CH_2(CH_2)_3CH_3$ | 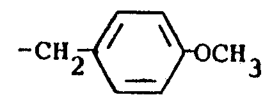 | H | |
| 157 | $-CH_2CH_2CH_3$ | $-CH_2CH_2CH_3$ | $-Ph$ | $-CH_2(CH_2)_2CH_3$ | H | |
| 158 | $-CH_2CH_2CH_3$ | $-CH_2CH_2CH_3$ | $-CH_2-Ph$ | $-CH_2(CH_2)_2CH_3$ | H | |
| 159 | $-(CH_2)_3CH_3$ | $-(CH_2)_3CH_3$ | $-CH_2CH_3$ | $-CH_2-Ph$ | H | |
| 160 | $-(CH_2)_3CH_3$ | $-(CH_2)_3CH_3$ | $-CH_2CH_2CH_3$ | $-CH_2-Ph$ | H | 115°C. |
| 161 | $-(CH_2)_3CH_3$ | $-(CH_2)_3CH_3$ | $-CH(CH_3)_2$ | $-CH_2-Ph$ | H | |
| 162 | $-(CH_2)_3CH_3$ | $-(CH_2)_3CH_3$ | $-(CH_2)_3CH_3$ | $-CH_2-Ph$ | H | 121°C. |
| 163 | $-(CH_2)_3CH_3$ | $-(CH_2)_3CH_3$ | $-CH_2CH(CH_3)_2$ | $-CH_2-Ph$ | H | |
| 164 | $-(CH_2)_3CH_3$ | $-(CH_2)_3CH_3$ | $-(CH_3)CHC_2H_5$ | $-CH_2-Ph$ | H | |
| 165 | $-(CH_2)_3CH_3$ | $-(CH_2)_3CH_3$ | $-CH_2(CH_2)_3CH_3$ | $-CH_2-Ph$ | H | |
| 166 | $-(CH_2)_3CH_3$ | $-(CH_2)_3CH_3$ | $-CH_2CH_2CH(CH_3)_2$ | $-CH_2-Ph$ | H | |
| 167 | $-(CH_2)_3CH_3$ | $-(CH_2)_3CH_3$ | $-CH_2(CH_2)_4CH_3$ | $-CH_2-Ph$ | H | |
| 168 | $-(CH_2)_3CH_3$ | $-(CH_2)_3CH_3$ | $-CH_2(CH_2)_5CH_3$ | $-CH_2-Ph$ | H | |
| 169 | $-(CH_2)_3CH_3$ | $-(CH_2)_3CH_3$ | $-CH_2(CH_2)_6CH_3$ | $-CH_2-Ph$ | H | |
| 170 | $-(CH_2)_3CH_3$ | $-(CH_2)_3CH_3$ | $-CH_3$ | 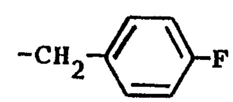 | H | 175°C. |

| No. | $R_1$ | $R_2$ | $R_3$ | $R_4$ | $R_5$ | m.p. |
|---|---|---|---|---|---|---|
| 171 | $-(CH_2)_3CH_3$ | $-(CH_2)_3CH_3$ | $-CH_2CH_2CH_3$ | 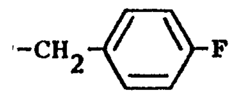 | H | 130.5°C. |
| 172 | $-(CH_2)_3CH_3$ | $-(CH_2)_3CH_3$ | $-(CH_2)_3CH_3$ | 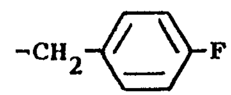 | H | 138°C. |
| 173 | $-(CH_2)_3CH_3$ | $-(CH_2)_3CH_3$ | $-CH_3$ | 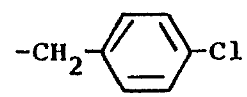 | H | |
| 174 | $-(CH_2)_3CH_3$ | $-(CH_2)_3CH_3$ | $-CH_2CH_2CH_3$ | 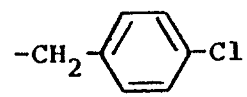 | H | |
| 175 | $-(CH_2)_3CH_3$ | $-(CH_2)_3CH_3$ | $-CH_3$ | 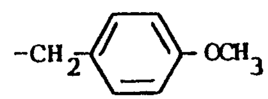 | H | 157°C. |

| No. | $R_1$ | $R_2$ | $R_3$ | $R_4$ | $R_5$ | m.p. |
|---|---|---|---|---|---|---|
| 176 | $—(CH_2)_3CH_3$ | $—(CH_2)_3CH_3$ | $—CH_2CH_3CH_3$ | 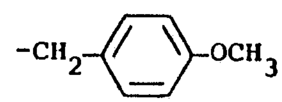 | H | 138°C. |
| 177 | $—(CH_2)_3CH_3$ | $—(CH_2)_3CH_3$ | —Ph | $—CH_2CH_2CH_3$ | H | |
| 178 | $—(CH_2)_3CH_3$ | $—(CH_2)_3CH_3$ | $—CH_2$—Ph | $—CH_2CH_2CH_3$ | H | |
| 179 | $—CH_2CH(CH_3)_2$ | $—CH_3$ | $—CH_2CH_3$ | $—CH_2$—Ph | H | |
| 180 | $—CH_2CH(CH_3)_2$ | $—CH_3$ | $—CH_2CH_2CH_3$ | $—CH_2$—Ph | H | |
| 181 | $—CH_2CH(CH_3)_2$ | $—CH_3$ | $—CH_2(CH_2)_2CH_3$ | $—CH_2$—Ph | H | |
| 182 | $—CH_2CH(CH_3)_2$ | $—CH_3$ | $—CH_2(CH_2)_3CH_3$ | $—CH_2$—Ph | H | |
| 183 | $—CH_2CH(CH_3)_2$ | $—CH_3$ | $—CH_3$ | $—CH_2$—Ph | H | |
| 184 | $—CH_2CH(CH_3)_2$ | $—CH_3$ | $—CH_3$ | —Ph | H | |
| 185 | $—CH_2CH(CH_3)_2$ | $—CH_3$ | $—CH_2CH_2CH_3$ | —Ph | H | |
| 186 | $—CH_2CH(CH_3)_2$ | $—CH_3$ | $—CH_2(CH_2)_2CH_3$ | —Ph | H | |
| 187 | $—CH_2CH(CH_3)_2$ | $—CH_3$ | —Ph | $—CH_2(CH_2)_2CH_3$ | H | |
| 188 | $—CH_2CH(CH_3)_2$ | $—CH_3$ | $—CH_2$—Ph | $—CH_2(CH_2)_2CH_3$ | H | |
| 189 | —Ph | $—CH_3$ | $—CH_2CH_2CH_3$ | $—CH_2$—Ph | H | |
| 190 | $—CH_3$ | —Ph | $—CH_2CH_2CH_3$ | $—CH_2$—Ph | H | |

| No. | $R_1$ | $R_2$ | $R_3$ | $R_4$ | $R_5$ | m.p. |
|---|---|---|---|---|---|---|
| 191 | $-CH_2-Ph$ | $-CH_3$ | $-CH_2CH_2CH_3$ | 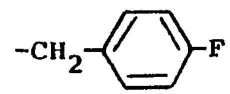 | H | |
| 192 | $-CH_3$ | $-CH_2Ph$ | $-CH_2CH_2CH_3$ | 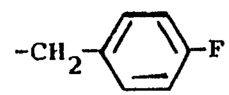 | H | |
| 193 | 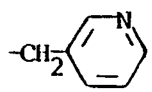 | $-CH_3$ | $-CH_2CH_2CH_3$ | $-CH_2-Ph$ | H | |
| 194 | $-CH_3$ | 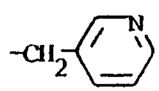 | $-CH_2CH_2CH_3$ | $-CH_2-Ph$ | H | |
| 195 | 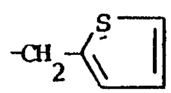 | $-CH_3$ | $-CH_2CH_2CH_3$ | $-CH_2-Ph$ | H | |

| No. | $R_1$ | $R_2$ | $R_3$ | $R_4$ | $R_5$ | m.p. |
|---|---|---|---|---|---|---|
| 196 | $-CH_3$ | 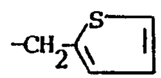 | $-CH_2CH_2CH_3$ | $-CH_2-Ph$ | H | |
| 197 | 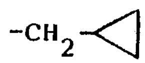 | $-CH_3$ | $-CH_2CH_2CH_3$ | $-CH_2-Ph$ | H | |
| 198 | $-CH_3$ | 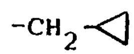 | $-CH_2CH_2CH_3$ | $-CH_2-Ph$ | H | |
| 199 | $-CH_2CH=CH_2$ | $-CH_3$ | $-CH_2CH_2CH_3$ | $-CH_2-Ph$ | H | |
| 200 | $-CH_3$ | $-CH_2CH=CH_2$ | $-CH_2CH_2CH_3$ | $-CH_2-Ph$ | H | |

| No. | $R_1$ | $R_2$ | $R_3$ | $R_4$ | $R_5$ | m.p. |
|---|---|---|---|---|---|---|
| 201 | $-CH_3$ | H | $-CH_2CH_2CH_3$ | $-CH_2-Ph$ | H | |
| 202 | H | $-CH_3$ | $-CH_2CH_2CH_3$ | $-CH_2-Ph$ | H | |
| 203 | H | H | $-CH_2CH_2CH_3$ | $-CH_2-Ph$ | H | |
| 204 | $-CH_2CH_3$ | $-CH_2CH_3$ | H | $-CH_2-Ph$ | H | |
| 205 | $-CH_2CH_2CH_3$ | $-CH_2CH_2CH_3$ | H | $-CH_2-Ph$ | H | |
| 206 | $-(CH_2)_3CH_3$ | $-(CH_2)_3CH_3$ | H | $-CH_2-Ph$ | H | |
| 207 | $-CH_3$ | $-CH_3$ | 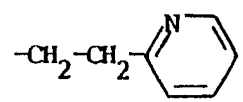 | $-CH_2-Ph$ | H | |
| 208 | $-CH_3$ | $-CH_3$ | 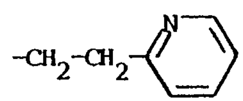 | 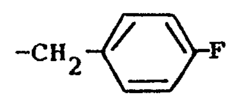 | H | |
| 209 | $-CH_3$ | $-CH_3$ | 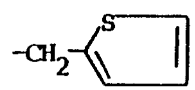 | $-CH_2-Ph$ | H | |
| 210 | $-CH_3$ | $-CH_3$ | $-CH_2CH_2-O-C_2H_5$ | $-CH_2-Ph$ | H | |

| No. | $R_1$ | $R_2$ | $R_3$ | $R_4$ | $R_5$ | m.p. |
|---|---|---|---|---|---|---|
| 211 | $-CH_3$ | $-CH_3$ | $-CH_2CH_2-S-C_2H_5$ | $-CH_2-Ph$ | H | |
| 212 | $-CH_3$ | $-CH_3$ | $-CH_2CH_2-S-n-C_3H_7$ | $-CH_2-Ph$ | H | |
| 213 | $-CH_3$ | $-CH_3$ | $-CH_2-S-n-C_4H_9$ | $-CH_2-Ph$ | H | |
| 214 | $-CH_3$ | $-CH_3$ | $-CH_2-O-n-C_4H_9$ | $-CH_2-Ph$ | H | |
| 215 | $-CH_3$ | $-CH_3$ | $-(CH_2)_3-CH_2-OH$ | $-CH_2-Ph$ | H | |
| 216 | $-CH_3$ | $-CH_3$ | $-CH_2CH_2-OH$ | $-CH_2-Ph$ | H | 183.5-184.5°C decomposes |
| 218 | $-CH_3$ | $-CH_3$ | $-CH_2-CH_2-CH_2-OH$ | $-CH_2-Ph$ | H | |
| 219 | $-CH_3$ | $-CH_3$ | OH<br>\|<br>$-CH_2-CH-CH_3$ | 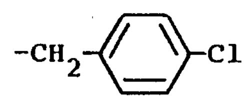 | H | |
| 220 | $-CH_3$ | $-CH_3$ | $-CH_2-CH_2-OH$ | 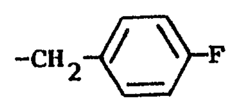 | H | |

| No. | $R_1$ | $R_2$ | $R_3$ | $R_4$ | $R_5$ | m.p. |
|---|---|---|---|---|---|---|
| 221 | $-CH_3$ | $-CH_3$ | $-CH_2-CH_2-CF_3$ | $-CH_2-Ph$ | H | |
| 222 | $-CH_3$ | $-CH_3$ | $-CH_2CH_2CH_3$ | 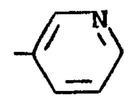 | H | |
| 223 | $-CH_3$ | $-CH_3$ | $-CH_2CH_2CH_3$ | 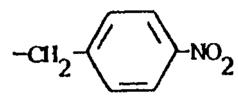 | H | |
| 224 | $-CH_3$ | $-CH_3$ | $-CH_2CH_2CH_3$ | 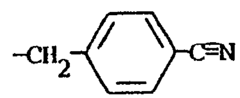 | H | |
| 225 | $-CH_3$ | $-CH_3$ | $-CH_2CH_2CH_3$ | 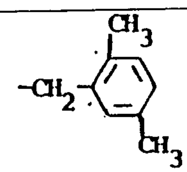 | H | |

| No. | $R_1$ | $R_2$ | $R_3$ | $R_4$ | $R_5$ | m.p. |
|---|---|---|---|---|---|---|
| 226 | $-CH_3$ | $-CH_3$ | $-CH_2CH_2CH_3$ | 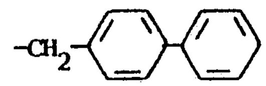 | H | |
| 227 | $-CH_3$ | $-CH_3$ | $-CH_2CH_2CH_3$ | 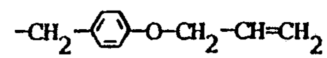 | H | |
| 228 | $-CH_3$ | $-CH_3$ | $-CH_2CH_2CH_3$ | 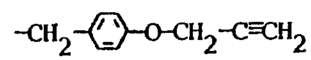 | H | |
| 229 | $-CH_3$ | $-CH_3$ | $-CH_2CH_2CH_3$ | 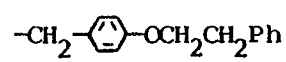 | H | |
| 230 | $-CH_3$ | $-CH_3$ | $-CH_2CH_2CH_3$ | 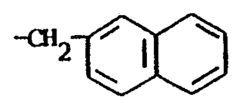 | H | |

| No. | $R_1$ | $R_2$ | $R_3$ | $R_4$ | $R_5$ | m.p. |
|---|---|---|---|---|---|---|
| 231 | $-CH_3$ | $-CH_3$ | $-CH_2CH_2CH_3$ | 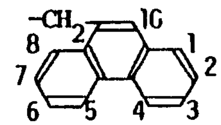 | H | |
| 232 | $-CH_3$ | $-CH_3$ | $-CH_2CH_2CH_3$ | 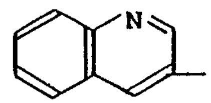 | H | |
| 233 | $-CH_3$ | $-CH_3$ | $-CH_2CH_2CH_3$ | 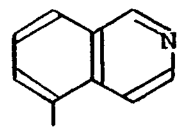 | H | |
| 234 | $-CH_3$ | $-CH_3$ | $-CH_2CH_2CH_3$ | 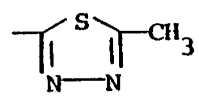 | H | |
| 235 | $-CH_3$ | $-CH_3$ | $-CH_2CH_2CH_3$ | 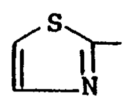 | H | |

| No. | $R_1$ | $R_2$ | $R_3$ | $R_4$ | $R_5$ | m.p. |
|---|---|---|---|---|---|---|
| 236 | $-CH_3$ | $-CH_3$ | $-CH_2CH_2CH_3$ | 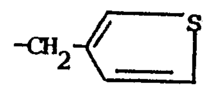 | H | |
| 237 | $-CH_3$ | $-CH_3$ | O<br>‖<br>$-CH_2CH_2OCCH_3$ | $-CH_2-Ph$ | H | 68°C (broad) |
| 238 | $-CH_3$ | $-CH_3$ | O<br>‖<br>$-CH_2CH_2CH_2OCCH_3$ | $-CH_2-Ph$ | H | |
| 239 | $-CH_3$ | $-CH_3$ | $OC(O)CH_3$<br>\|<br>$-CH_2CHCH_3$ | 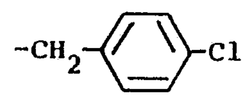 | H | |
| 240 | $-CH_3$ | $-CH_3$ | O<br>‖<br>$-CH_2CH_2OCCH_3$ | 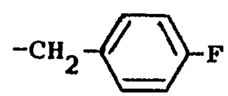 | H | |

| No. | $R_1$ | $R_2$ | $R_3$ | $R_4$ | $R_5$ | m.p. |
|---|---|---|---|---|---|---|
| 241 | $-CH_3$ | $-CH_3$ | $-CH_2CH_2CH_3$ | 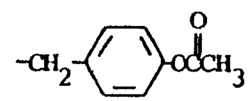 | H | |
| 242 | $-CH_3$ | $-CH_3$ | $-CH_2CH_2CH_3$ | 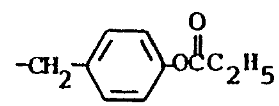 | H | |
| 243 | $-CH_3$ | $-CH_3$ | $-CH_2CH_2CH_3$ | 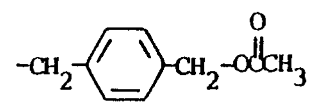 | H | |
| 244 | $-CH_3$ | $-CH_3$ | $-CH_2CH_2CH_3$ | 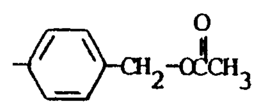 | H | |
| 245 | $-CH_3$ | $-CH_3$ | $-CH_3$ | 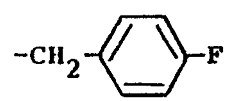 | $CH_3C(O)-$ | (.1/2 $H_2O$) 228—230.5°C |

| No. | $R_1$ | $R_2$ | $R_3$ | $R_4$ | $R_5$ | m.p. |
|---|---|---|---|---|---|---|
| 246 | $—CH_3$ | $—CH_3$ | $—CH_2CH_2CH_3$ | $—CH_2—Ph$ | $CH_3C(O)—$ | |
| 247 | $—CH_3$ | $—CH_3$ | $—CH_2CH_2CH_3$ | $—CH_2—Ph$ | $n\text{-}C_4H_9—$ | |
| 248 | $—CH_3$ | $—CH_3$ | $—CH_3$ | 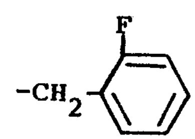 | H | 224—226°C |

| No. | $R_1$ | $R_2$ | $R_3$ | $R_4$ | $R_5$ | m.p. |
|---|---|---|---|---|---|---|
| 249 | $—CH_3$ | $—CH_3$ | $—CH_2CH_2—H_3$ | 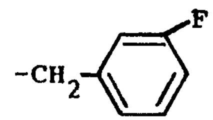 | H | 213—214.5°C |
| 250 | $—CH_3$ | $—CH_3$ | $—CH_3$ | 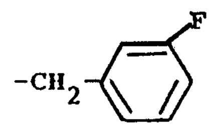 | H | .1/4 $H_2O$<br>218.5—219.5°C |
| 251 | $—CH_3$ | $—CH_3$ | $—CH_2CH_2CH_3$ | $CH_3$<br>\|<br>—CH—Ph | H | 197—200°C |
| 252 | $—CH_3$ | $—CH_3$ | $CH_3$<br>/<br>$—CH_2—CH_2—N$<br>\\<br>$CH_3$ | $—CH_2—Ph$ | H | .HCl<br>195—197°C dec. |
| 253 | $—CH_3$ | $—CH_3$ | $—CH_2—CH=CH_2$ | $—CH_2—Ph$ | H | 222—224°C |

| No. | $R_1$ | $R_2$ | $R_3$ | $R_4$ | $R_5$ | m.p. |
|---|---|---|---|---|---|---|
| 254 | $-CH_3$ | $-CH_3$ | $-CH_2C \equiv CH$ | $-CH_2-Ph$ | H | 236—238°C |
| 255 | $-CH_3$ | $-CH_3$ | $CH_3$<br>$-CH_2-CH=C$<br>$CH_3$ | $-CH_2-Ph$ | H | 186—188°C |
| 256 | $-CH_3$ | $-CH_3$ | $-CH_2-CH=CH-CH_3$ | $-CH_2-Ph$ | H | 206—208°C |
| 257 | $-CH_3$ | $-CH_3$ | $-CH_2-Ph$ | $-CH_2-Ph$ | H | 190—195°C |
| 258 | $-CH_3$ | $-CH_3$ | $-(CH_2)_3CH_3$ | 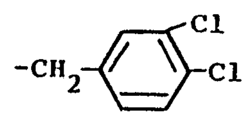 | H | 214—216°C |

| No. | $R_1$ | $R_2$ | $R_3$ | $R_4$ | $R_5$ | m.p. |
|---|---|---|---|---|---|---|
| 259 | $-CH_3$ | $-CH_3$ | $-(CH_2)_3CH_3$ | 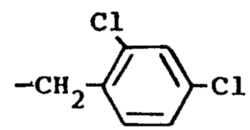 | H | 220—222°C |
| 260 | $-CH_3$ | $-CH_3$ | $-(CH_2)_3CH_3$ | 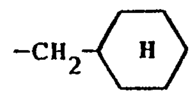 | H | 151—153°C |
| 261 | $-CH_3$ | $-CH_3$ | 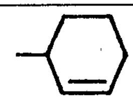 | $-CH_2-Ph$ | H | 157—161°C |
| 262 | $-CH_3$ | $-CH_3$ | $-CH_2C \equiv N$ | $-CH_2-Ph$ | H | dec. >195°C |
| 263 | $-CH_3$ | $-CH_3$ | $-CH_2C(=O)-OCH_3$ | $-CH_2-Ph$ | H | 211—214.5°C |

| No. | $R_1$ | $R_2$ | $R_3$ | $R_4$ | $R_5$ | m.p. |
|---|---|---|---|---|---|---|
| 264 | $—CH_3$ | $—CH_3$ | $—CH_2\overset{\overset{O}{\|}}{C}OH$ | $—CH_2—Ph$ | H | .3/4 $H_2O$ 258—258.5°C (decomposes) |
| 265 | $—CH_3$ | $—CH_3$ | $—CH_3$ | 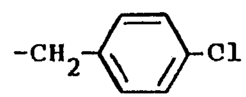 | H | 236—238°C |
| 266 | $—(CH_2)_3CH_3$ | $—(CH_2)_3CH_3$ | $—(CH_2)_3CH_3$ | 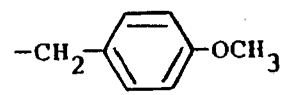 | H | 129—131°C |
| 267 | $—(CH_2)_3CH_3$ | $—(CH_2)_3CH_3$ | $—CH_3$ | 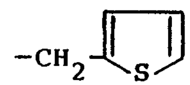 | H | 100.5—102°C |
| 268 | $—(CH_2)_3CH_3$ | $—(CH_2)_3CH_3$ | $—CH_2CH_2CH_3$ | 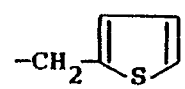 | H | 115—117°C |

| No. | $R_1$ | $R_2$ | $R_3$ | $R_4$ | $R_5$ | m.p. |
|---|---|---|---|---|---|---|
| 269 | $-(CH_2)_3CH_3$ | $-(CH_2)_3CH_3$ | $-CH_2CH_2CH(CH_3)_2$ | 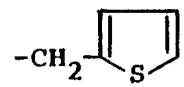 | H | 133.5—135.0°C |
| 270 | $-CH_3$ | $-CH_3$ | $-CH_3$ | $-CH_2CH_2-Ph$ | H | 222—224°C |
| 271 | $-CH_3$ | $-CH_3$ | 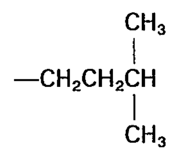 | $-CH_2CH_2-Ph$ | H | 197—198.5°C |
| 272 | $-(CH_2)_3CH_3$ | $-(CH_2)_3CH_3$ | $-CH_2CH_2CH_3$ | $-CH_2CH_2-Ph$ | H | 117—118.5°C |
| 273 | $-(CH_2)_3CH_3$ | $-(CH_2)_3CH_3$ | $-(CH_2)_3CH_3$ | $-CH_2CH_2-Ph$ | H | 81—83°C |
| 274 | $-CH_3$ | $-CH_3$ | $-CH_2CH_2CH_3$ | $-CH_2CH_2-Ph$ | H | 168—170°C |
| 275 | $-CH_3$ | $-CH_3$ | 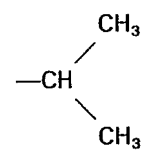 | $-CH_2CH_2-Ph$ | H | 209—211°C |
| 276 | $-(CH_2)_3CH_3$ | $-(CH_2)_3CH_3$ | $-CH_3$ | $-CH_2CH_2-Ph$ | H | 126—128°C |
| 277 | $-CH_3$ | $-CH_3$ | $-CH_2CH_2CH_3$ | $-CH_2(CH_2)_4CH_3$ | $Na^+$ | 245—260°C |
| 278 | $-CH_3$ | $-CH_3$ | $-H$ | $-CH_2-Ph$ | $Na^+$ | $.H_2O$ >295°C |

| No. | $R_1$ | $R_2$ | $R_3$ | $R_4$ | $R_5$ | m.p. |
|---|---|---|---|---|---|---|
| 279 | $-CH_3$ | $-CH_3$ | $-CH_2CH_2CH_3$ | $-CH_2-Ph$ | $CH_3$ | 199—201°C |
| 280 | $-CH_3$ | $-CH_3$ | $-H$ | 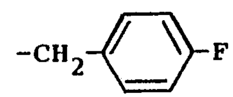 | $Na^+$ | $.H_2O$ 260—280°C |
| 281 | $-CH_3$ | $-CH_3$ | 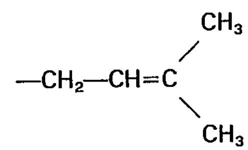 | 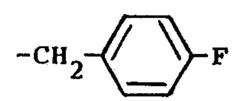 | $Na^+$ | $.H_2O$ 260—280°C |
| 282 | $-CH_3$ | $-CH_3$ | $-CH_2-CH=CH_2$ | $-CH_2-Ph$ | $Na^+$ | 280—300°C |
| 283 | $-CH_3$ | $-CH_3$ | $-CH_2-CH=CH-CH_3$ | $-CH_2-Ph$ | $Na^+$ | 260—280°C |

| No. | $R_1$ | $R_2$ | $R_3$ | $R_4$ | $R_5$ | m.p. |
|---|---|---|---|---|---|---|
| 284 | $-CH_3$ | $-CH_3$ | 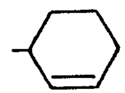 | $-CH_2-Ph$ | $Na^+$ | 280—300°C |
| 285 | $-CH_3$ | $-CH_3$ | $-CH_2-\overset{O}{\overset{\|}{C}}-OCH_3$ | $-CH_2-Ph$ | $Na^+$ | 227—230°C (decomposes) |
| 286 | $-CH_3$ | $-CH_3$ | $-CH_2CH=C(CH_3)_2$ | 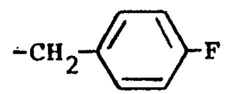 | H | 232—234°C |
| 287 | $-CH_3$ | $-CH_3$ | $-CH_2CH=C(CH_3)_2$ | 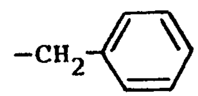 | $Na^+$ | 240—260°C |
| 288 | $-CH_3$ | $-CH_3$ | $-CH_2CH_2OCH_2CH_3$ | 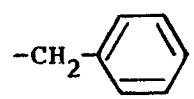 | $Na^+ \cdot 3/4\ H_2O$ | >300°C |
| 289 | $-CH_3$ | $-CH_3$ | $-CH_2CH_2SCH_2CH_3$ | 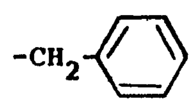 | $Na^+ \cdot 1/4\ H_2O$ | 298—300°C (decomposes) |
| 290 | $-CH_3$ | $-CH_3$ | $-CO_2C_2H_5$ | 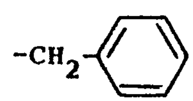 | $Na^+ \cdot H_2O$ | >240°C (decomposes) |

The anti-inflammatory potential of the compounds of the present invention may be determined by the Reversed Passive Arthus Reaction (RPAR) Synovitis technique as set forth below using male Lewis rats (obtained from Charles River Breeding Laboratories) weighing 200—250 g. The potency of the compounds is determined using indomethacin as the standard. On the basis of the test results, a dosage range of about 0.1 milligrams per kilogram of body weight per day to about 50 milligrams per kilogram of body weight per day in divided doses at about 4 hour intervals is recommended. For the preferred compounds of the invention tested, it is considered that the compounds are effectively non-toxic at the dose-rates indicated for treatment of inflammatory conditions.

Of course, the dosage to be administered depends upon the particular compound use, the age and general health of the patient and the severity of the inflammatory condition. Thus, the dose ultimately decided upon must be left to the judgement of a trained health-care practitioner.

RPAR Synovitis Technique:

A Lewis rat is dosed orally with drug or placebo one hour prior to intravenous administration of 2.28 mg of bovine serum albumin (BSA) in 0.2 cc of pyrogen free saline followed by the intraarticular injection of 0.54 mg of rabbit anti-BSA antibody in 0.03 cc of pyrogen free saline into one knee joint. The contralateral knee is injected with 0.03 cc of pyrogen free saline. All injections are made with the animal under light ether anaesthesia. Three hours later the rat is again dosed orally with drug or placebo. All drug doses are split. That is, one-half of the dose is administered before lesion induction and one-half is administered after lesion induction.

The following morning (about 17 hours after lesion induction) the rat is killed and both knee joints are exposed. The subpatellar areolar tissue with attendant synovium is excised and weighed. Differences between the weight of antibody and saline injected knees are considered to represent the inflammatory response for each animal (delta synovial weight). Differences in delta synovial weight between lesion controls and drug-treated rats are evaluated for statistical significance with an analysis of variance. Relative potencies are determined with a linear regression analysis.

For a dosage of 50 mpk of tested compounds the percentage inhibition of inflammation (I) listed in the following table (Table III) has been observed:

TABLE III

| Compound | I |
|---|---|
| A | 46 |
| B | 48 |

A: 9-benzyl-1,3-dimethyl-8-hydroxy-7-n-propyl-9*H*-6-oxo-pyridmido[2,1-f]purine-2,4-dione (sodium salt),

B: 9-benzyl-1,3-dimethyl-8-hydroxy-7-(n-butyl)-*H*-6-oxo-pyrimido[2,1-f]purine-2,4-dione.

Moreover, compound A has been tested and found not to be ulcerogenic at dosages as high as 100 mg per kg (oral administration).

The compounds of this invention may be processed and dispensed in tablets, capsules or elixirs, for oral administration; and solutions or suspensions for parenteral administration. In whatever form the compounds are dispensed, they may be admixed with the pharmaceutically acceptable excipients, binders, dispersing agents and carriers generally used in the art.

Exemplary of the pharmaceutical carriers, excipients, preservatives and binders are gelatin, lactose, starch, magnesium stearate, talc, vegetable oils, gums and polyalkylene glycols. The pharmaceutical dosage forms are prepared by the methods conventionally used in the art. Further, the dosage units may also contain a compatible anti-depressant and/or analgesics to treat the depression and pain usually associated with chronic inflammatory conditions.

In the following formulation examples the following compounds of the invention are used:

9-benzyl-1,3-dimethyl-8-hydroxy-7-n-propyl-9*H*-6-oxopyrimido[2,1-f]purine-2,4-dione,
9-(4'-fluorobenzyl)-6-hydroxy-1,3-dimethyl-7-n-propyl-9*H*-8-oxo-pyrimidio[2,1-f]purine-2,4-dione,
9-benzyl-1,3-dimethyl-8-hydroxy-7-(n-butyl)-9*H*-6-oxo-pyrimido[2,1-f]purine-2,4-dione,
9-(4-fluorobenzyl)-1,3-dimethyl-6-hydroxy-7-(n-butyl)-9*H*-6-oxo-pyrimido[2,1-f]purine-2,4-dione,
9-benzyl-1,3-dimethyl-6-hydroxy-7-(3-methyl-2-butenyl)pyrimido[2,1-f]purine-2,4,8(1,*H*,3*H*,9*H*)trione

or

sodium salt of 1,3-dimethyl-9-(4-fluorobenzyl)-6-hydroxy-7-(3-methyl-2-butenyl)-pyrimido[2,1-f]-purine-2,4,8(1*H*,3*H*,9*H*)trione.

Formulation 1

Capsules:

| No. | Ingredient | | mg/capsule | mg/capsule |
|---|---|---|---|---|
| 1. | Compound of the invention | | 50 | 250 |
| 2. | Lactose USP | | 50 | 100 |
| 3. | Corn Starch, Food Grade | | 48.5 | 50 |
| 4. | Microcrystalline Cellulose NF | | 50 | 95 |
| 5. | Magnesium Stearate NF | | 1.5 | 5 |
| | | Total | 200 | 500 |

Method of Manufacture

Mix Item Nos. 1, 2, 3 and 4 in a suitable mixer 10—15 minutes. Add Item No. 5 and mix for 1—3 minutes. Fill the mixture into suitable two-piece hard gelatin capsules using encapsulating machine.

Formulation 2

Tablets:

| No. | Ingredient | | mg/capsule | mg/capsule |
|---|---|---|---|---|
| 1. | Compound of the invention | | 50 | 250 |
| 2. | Lactose USP | | 68 | 57 |
| 3. | Corn Starch, Food Grade, as a 10% paste in Purified Water | | 10 | 20 |
| 4. | Corn Starch, Food Grade | | 20 | 18 |
| 5. | Magnesium Stearate NF | | 2 | 5 |
| | | Total | 150 | 350 |

Method of Manufacture

Mix Items Nos. 1 and 2 in a suitable mixer for 10—15 minutes. Granulate the mixture with Item No. 3. Pass the wet granulation through a coarse screen [e.g., 6.3 mm (1/4")] if needed, and dry the wet granules. Mill the dried granules. Combine Item No. 4 and the dried granules and mix for 10—15 minutes. Add Item No. 5 and mix for 1—3 minutes. Compress the mixture to appropriate size and weight on a suitable tablet machine.

**Claims for the Contracting States: BE CH DE FR GB IT LI LU NL SE**

1. A compound of the general formula (I)

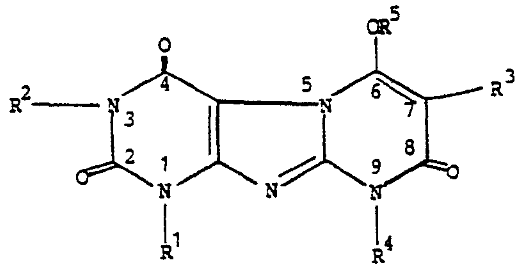

(I)

and its tautomer and the pharmaceutically acceptable salts thereof, wherein

$R^1$ and $R^2$ are the same or different and are hydrogen, alkyl having 1 to 8 carbon atoms, cycloalkyl having 3 to 8 carbon atoms, alkenyl having 3 to 8 carbon atoms, alkynyl having 3 to 8 carbon atoms, aryl such as phenyl, naphthyl, phenanthryl, or substituted phenyl;

alkyl (having 1 to 8 carbon atoms) substituted by cycloalkyl (having 3 to 8 carbon atoms) aryl as defined above or heterocyclic radical selected from quinolinyl, isoquinolinyl, pyridinyl, thiazolyl, 1,3,4-thiadiazolyl, and thiophenyl;

each of the aforementioned substituted phenyl and substituted heterocyclic radicals may be substituted with one to three radicals that are independently selected from halogen (i.e., fluoro, chloro, bromo, iodo), trifluoromethyl, nitro, cyano, $C_1$ to $C_6$ alkoxy,
$C_1$ to $C_6$ alkyl, phenyl, hydroxy, $C_1$ to $C_6$ alkylthio,

$$-\overset{O^{-}}{\overset{|}{S^{+}}}-(C_1 \text{ to } C_6 \text{ alkyl}),$$

$-SO_2-(C_1$ to $C_6$ alkyl), $-O-(C_3$ to $C_0$ alkenyl), $-O-(C_3$ to $C_8$ alkynyl), $-OC_mH_{2m}$-phenyl wherein m is an integer from 0 to 4, $C_1$ to $C_6$ acyloxy, hydroxy-($C_1$ to $C_6$ alkyl), ($C_1$ to $C_6$ acyloxy)-($C_1$ to $C_6$ alkyl)-, cyano-($C_1$ to $C_6$ alkyl), $-CONH_2$, $-CO_2H$, and -($C_1$ to $C_4$ alkyl)-$CO_2$($C_1$ to $C_4$ alkyl);

$R^3$ is hydrogen, alkyl containing 1 to 8 carbon atoms, cycloalkyl containing 3 to 8 carbon atoms, alkenyl containing 2 to 8 carbon atoms, cycloalkenyl containing 5 to 8 carbon atoms, alkynyl containing 3 to 8 carbon atoms, acyloxyalkyl containing 2 to 12 carbon atoms, oxoalkyl containing 1 to 8 carbon atoms, aryl as defined above;

alkyl (containing 1 to 8 carbon atoms) substituted by cycloalkyl (containing 3 to 8 carbon atoms), cycloalkenyl (containing 5 to 8 carbon atoms), aryl as defined above, hydroxy, cyano, halo or heterocyclic radicals as defined above;

-alkyl-X-$C_nH_{2n+1}$ wherein alkyl contains 1 to 8 carbon atoms, n is an integer from 0 to 4 and X is O,S,$S^+-O^-$, $SO_2$ or $-N-C_pH_{2p+1}$ wherein p is an integer from 0 to 4;

$-(CH_2)_q\,C(O)NR^6R^7$ (wherein $R^6$ and $R^7$ are independently hydrogen or alkyl having from 1 to 8 carbon atoms, and q is an integer from 0 to 6), $-(CH_2)_rC(O)OR^8$ (wherein $R^8$ is hydrogen or alkyl having from 1 to 8 carbon atoms and r is an integer from 0 to 6);

$R^4$ is hydrogen, alkyl containing 1 to 8 carbon atoms, aryl as defined above, a heterocyclic radical as defined above;

alkyl (containing 1 to 8 carbon atoms) substituted by cycloalkyl (containing 3 to 8 carbon atoms), aryl as defined above or a heterocyclic radical as defined above;

$R^5$ is hydrogen, acyl containing 2 to 8 carbon atoms or alkyl containing 1 to 4 carbon atoms.

2. A compound according to claim 1, wherein $R^1$ and $R^2$ are the same or different and are alkyl having 1 to 8 carbon atoms, cycloalkyl having 3 to 8 carbon atoms, alkenyl having 3 to 8 carbon atoms, phenyl;

alkyl (having 1 to 8 carbon atoms) substituted by cycloalkyl (having 3 to 8 carbon atoms), phenyl, pyridyl or thienyl;

$R^3$ is hydrogen, alkyl containing 1 to 8 carbon atoms, cycloalkyl containing 3 to 8 carbon atoms, alkenyl containing 2 to 8 carbon atoms, cycloalkenyl containing 5 to 8 carbon atoms, alkynyl containing 3 to 8 carbon atoms, acyloxyalkyl containing 2 to 12 carbon atoms, phenyl;

alkyl (containing 1 to 8 carbon atoms) substituted by phenyl, hydroxy, cyano, halo, pyridyl or thienyl;

-alkyl-X$-C_nH_{2n+1}$ wherein alkyl contains 1 to 8 carbon atoms, n is an integer from 0 to 4 and X is O, S or $-N-C_pH_{2p+1}$ wherein p is an integer from 0 to 4;

$-(CH_2)_rC(O)OR^8$ (wherein $R^8$ is hydrogen or alkyl having from 1 to 8 carbon atoms and r is an integer from 0 to 6);

$R^4$ is alkyl containing 1 to 8 carbon atoms, phenyl, pyridyl or thienyl;

alkyl (containing 1 to 8 carbon atoms) substituted by cycloalkyl (containing 3 to 8 carbon atoms), phenyl, pyridyl or thienyl;

$R^5$ is hydrogen, acyl containing 2 to 8 carbon atoms or alkyl containing 1 to 4 carbon atoms.

3. A compound according to claim 1 or 2, wherein $R^1$ and $R^2$ are the same or different and are alkyl having 1 to 4 carbon atoms;

$R^3$ is hydrogen, alkyl containing 1 to 5 carbon atoms, alkenyl containing 3 to 5 carbon atoms, cyclohexenyl, alkynyl containing 3 carbon atoms, acyloxyalkyl containing 4 carbon atoms, phenyl;

alkyl (containing 1 or 2 carbon atoms) substituted by phenyl, hydroxy or cyano;

$-CH_2-CH_2-N-(CH_3)_2$, $-CH_2C(O)OH$, $-CH_2C(O)OCH_3$, $-CH_2CH_2OCH_2CH_3$, $-CH_2CH_2SCH_2CH_3$ or $-CO_2C_2H_5$;

$R^4$ is alkyl containing 1 to 6 carbon atoms, phenyl or fluoro-substituted phenyl;

alkyl (containing 1 to 2 carbon atoms) substituted by cyclohexyl, phenyl (unsubstituted or substituted by fluoro, chloro, or methoxy), thienyl or pyridyl;

$R^5$ is hydrogen, acetyl or methyl.

4. A compound according to any one of claims 1 to 3, wherein $R^1$ and $R^2$ are as defined above, $R^3$ is n-propyl, n-butyl, or 3-methyl-2-butenyl, $R^4$ is benzyl or p-fluorobenzyl and $R^5$ is hydrogen.

5. A compound according to any one of claims 1 to 4, wherein $R^1$ and $R^2$ are methyl.

6. A compound according to any one of claims 1 to 5 in the form of its sodium salt, wherein $R^5$ is replaced by $Na^+$.

7. A compound according to any one of claims 1 to 6, which is

9-benzyl-1,3-dimethyl-8-hydroxy-7-n-propyl-9*H*-6-oxopyrimido[2,1-f]purine-2,4-dione,
9-(4'-fluorobenzyl)-6-hydroxy-1,3-dimethyl-7-n-propyl-9*H*-8-oxo-pyrimido[2,1-f]purine-2,4-dione,
9-benzyl-1,3-dimethyl-8-hydroxy-7-(n-butyl)-9*H*-6-oxo-pyrimido[2,1-f]purine-2,4-dione,
9-(4-fluorobenzyl)-1,3-dimethyl-6-hydroxy-7-(n-butyl)-9*H*-8-oxo-pyrimido[2,1-f]purine-2,4-dione,
9-benzyl-1,3-dimethyl-6-hydroxy-7-(3-methyl-2-butenyl)pyrimido[2,1-f]purine-2,4,8(1,*H*,3*H*,9*H*)trione
or
sodium salt of 1,3-dimethyl-9-(4-fluorobenzyl)-6-hydroxy-7-(3-methyl-2-butenyl)-pyrimido[2,1-f]-purine-2,4,8(1*H*,3*H*,9*H*)trione.

8. Process for the preparation of compounds defined in any one of claims 1 to 7, characterized in that the compounds are prepared by an appropriate process or combination of processes selected from the following processes

a) for the preparation of compounds as defined in claim 1, wherein $R^5$ is hydrogen and $R^1$, $R^2$, $R^3$ and $R^4$ are as defined in claim 1: reaction of an 8-aminoxanthine of formula II

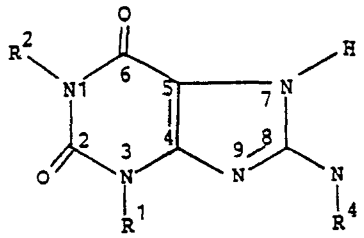

II

with a reactive derivative of $R^3$-substituted malonic acid;

b) for the preparation of compounds of formula I, wherein $R^1$, $R^2$ and $R^4$ are as defined above, $R^5$ is hydrogen and $R^3$ is methyl, ethyl, propyl, alkenyl containing 3 to 8 carbon atoms, cycloalkenyl containing 5 to 8 carbon atoms, alkynyl containing 3 to 8 carbon atoms;

alkyl (containing 1 to 8 carbon atoms) substituted by cyclopropyl, cycloalkenyl (containing 5 to 8 carbon atoms), aryl as defined above or cyano; or

$-CH_2-C(O)OR^8$ (wherein $R^8$ is alkyl having from 1 to 8 carbon atoms);

whereby in the groups alkenyl, cycloalkenyl, alkynyl and substituted alkyl the carbon atom being in α-position to the unsaturation of the —CN group or aryl as defined above respectively, is connected with the carbon atoms of position 7 of the pyrimido[2,1-f]purine (I):

reaction of a compound of formula XII or a sodium salt thereof, wherein $R^1$, $R^2$, $R^4$ and $R^5$ are defined as above, in the presence of sodium hydride in an aprotic organic solvent with a compound hal-$R^3$ (XIII), wherein hal is halogen,

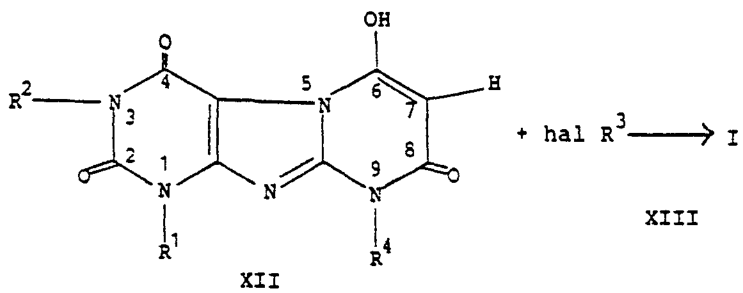

XII

XIII

whereby when $R^3$ is alkenyl, cycloalkenyl, alkynyl or substituted alkyl the halogen is in 2-position to the unsaturation or the —CN group or aryl as defined above respectively;

c) for the preparation of compounds of formula I, wherein $R^1$ and $R^2$ are the same or different and are hydrogen, alkyl having 1 to 8 carbon atoms, cycloalkyl having 4 to 8 carbon atoms, aryl as defined above;

alkyl (having 1 to 8 carbon atoms) substituted by cycloalkyl (having 4 to 8 carbon atoms), aryl as defined above or heterocyclic radical as defined above;

$R^3$ is alkyl containing 2 to 8 carbon atoms, cycloalkyl containing 5 to 8 carbon atoms;

$R^4$ is hydrogen, alkyl containing 1 to 8 carbon atoms, aryl as defined above, a heterocyclic radical as defined above;

alkyl (containing 1 to 8 carbon atoms) substituted by cycloalkyl (containing 4 to 8 carbon atoms), aryl as defined above or a heterocyclic radical as defined above;

$R^5$ is hydrogen, acyl containing 2 to 8 carbon atoms or alkyl containing 1 to 4 carbon atoms:

hydrogenation of corresponding compounds of formula I, wherein $R^1$, $R^2$, $R^4$ and $R^5$ are defined as above and $R^3$ is alkenyl containing 2 to 8 carbon atoms, cycloalkenyl containing 5 to 8 carbon atoms or alkynyl containing 3 to 8 carbon atoms;

followed if desired by one or more aftertreatments

i) alkylation of compound of formula I wherein $R^5$ is hydrogen to form the corresponding compound wherein $R^5$ is alkyl;

ii) esterification of a compound of formula I, wherein $R^3$ is alkyl substituted by hydroxy to form the corresponding acyloxyalkyl-compound;

iii) salt formation;

iv) reduction of a compound of formula I, wherein $R^7$ is $-(CH_2)_rC(O)OR^8$ to the corresponding compound wherein $R^3$ is $-(CH_2)_rCH_2OH$.

9. Process according to claim 8a, characterized in that for the preparation of compounds (I) wherein $R^3$ is hydrogen, alkyl, cycloalkyl, alkyl substituted by cycloalkyl, and -alkyl-X—$C_nH_{2n+1}$, wherein X —N—$C_pH_{2p+1}$ the 8-aminoxanthine (II) is reacted with a diester of the $R^3$-containing malonic acid in the presence of a base such as sodium methoxide at elevated temperature.

10. Process according to claim 8a, characterized in that for the preparation of compounds wherein $R^3$ is hydrogen, the 8-aminoxanthine (II) is reacted with a half-ester of the $R^3$-containing malonic acid halogenide in a suitable solvent at elevated temperature.

11. Process according to claim 8a, characterized in that the 8-aminoxanthine (II) is reacted with a di-ester of the $R^3$-containing malonic acid and a stoichiometric amount of a base (e.g. sodium hydride) in an organic solvent.

12. A pharmaceutical composition comprising a compound as defined in any one of claims 1 to 7 or obtained according to a process of any one of claims 8 to 11, if desired in the form of a shaped dosage unit.

13. Process for preparing a pharmaceutical composition characterized in that a compound as defined in any one of claims 1 to 7 or obtained according to a process of any one of claims 8 to 11 is brought into a form suitable for therapeutic administration.

**Claims for the Gontracting State: AT**

1. Process for the preparation of compounds of the general formula (I)

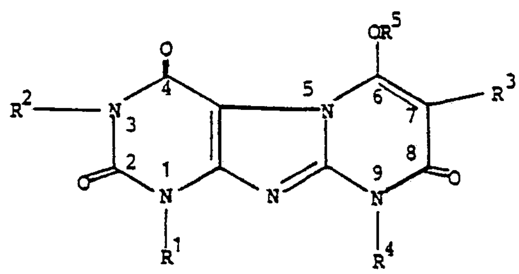

(I)

their tautomers and the pharmaceutically acceptable salts thereof, wherein

$R^1$ and $R^2$ are the same or different and are hydrogen, alkyl having 1 to 8 carbon atoms, cycloalkyl having 3 to 8 carbon atoms, alkenyl having 3 to 8 carbon atoms, alkynyl having 3 to 8 carbon atoms, aryl such as phenyl, naphthyl, phenanthryl, or substituted phenyl;

alkyl (having 1 to 8 carbon atoms) substituted by cycloalkyl (having 3 to 8 carbon atoms) aryl as defined above or heterocyclic radical selected from quinolinyl, isoquinolinyl, pyridinyl, thiazolyl, 1,3,4-thiadiazolyl, and thiophenyl;

each of the aforementioned substituted phenyl and substituted heterocyclic radicals may be substituted with one to three radicals that are independently selected from halogen (i.e., fluoro, chloro, bromo, iodo), trifluoromethyl, nitro, cyano, $C_1$ to $C_6$ alkoxy,
$C_1$ to $C_6$ alkyl, phenyl, hydroxy, $C_1$ to $C_6$ alkylthio,

$$-\overset{O^-}{\overset{|}{S^+}}-(C_1 \text{ to } C_6 \text{ alkyl}),$$

$-SO_2-(C_1$ to $C_6$ alkyl), $-O-(C_3$ to $C_8$ alkenyl), $-O-(C_3$ to $C_8$ alkynyl), $-OC_mH_{2m}$-phenyl wherein m is an integer from 0 to 4, $C_1$ to $C_6$ acyloxy, hydroxy-($C_1$ to $C_6$ alkyl), ($C_1$ to $C_6$ acyloxy)-($C_1$ to $C_6$ alkyl)-, cyano-($C_1$ to $C_6$ alkyl), $-CONH_2$, $-CO_2H$, and -($C_1$ to $C_4$ alkyl)-$CO_2$($C_1$ to $C_4$ alkyl);

$R_3$ is hydrogen, alkyl containing 1 to 8 carbon atoms, cycloalkyl containing 3 to 8 carbon atoms, alkenyl containing 2 to 8 carbon atoms, cycloalkenyl containing 5 to 8 carbon atoms, alkynyl containing 3 to 8 carbon atoms, acyloxyalkyl containing 2 to 12 carbon atoms, oxoalkyl containing 1 to 8 carbon atoms, aryl as defined above;

alkyl (containing 1 to 8 carbon atoms) substituted by cycloalkyl (containing 3 to 8 carbon atoms), cyclalkenyl (containing 5 to 8 carbon atoms), aryl as defined above, hydroxy, cyano, halo or heterocyclic radicals as defined above;

-alkyl-X-$C_nH_{2n+1}$ wherein alkyl contains 1 to 8 carbon atoms, n is an integer from 0 to 4 and X is O,S,S,$^+$—$O^-$, $SO_2$ or —N—$C_pH_{2p+1}$ wherein p is an integer from 0 to 4;

$-(CH_2)_q$ $C(O)NR^6R^7$ (wherein $R^6$ and $R^7$ are independently hydrogen or alkyl having from 1 to 8 carbon

atoms, and q is an integer from 0 to 6), $-(CH_2)_rC(O)OR^8$ (wherein $R^8$ is hydrogen or alkyl having from 1 to 8 carbon atoms and r is an integer from 0 to 6);

$R^4$ is hydrogen, alkyl containing 1 to 8 carbon atoms, aryl as defined above, a heterocyclic radical as defined above;

alkyl (containing 1 to 8 carbon atoms) substituted by cycloalkyl (containing 3 to 8 carbon atoms), aryl as defined above or a heterocyclic radical as defined above;

$R^5$ is hydrogen, acyl containing 2 to 8 carbon atoms or alkyl containing 1 to 4 carbon atoms,

characterized in that the compounds are prepared by an appropriate process or combination of processes selected from the following processes

a) for the preparation of compounds as defined above, wherein $R^5$ is hydrogen and $R^1$, $R^2$, $R^3$ and $R^4$ are as defined in claim 1: reaction of an 8-aminoxanthine of formula II

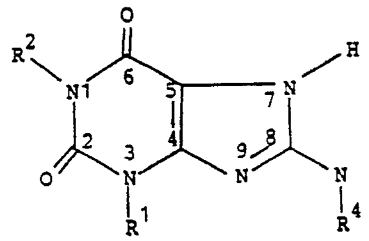

II

with a reactive derivative of $R^3$-substituted malonic acid;

b) for the preparation of compounds of formula I, wherein $R^1$, $R^2$ and $R^4$ are as defined above, $R^5$ is hydrogen and $R^6$ is methyl, ethyl, propyl, alkenyl containing 3 to 8 carbon atoms, cycloalkenyl containing 5 to 8 carbon atoms, alkynyl containing 3 to 8 carbon atoms;

alkyl (containing 1 to 8 carbon atoms) substituted by cyclopropyl, cycloalkenyl (containing 5 to 8 carbon atoms), aryl as defined above or cyano; or

$-CH_2-C(O)OR^8$ (wherein $R^8$ is alkyl having from 1 to 8 carbon atoms);

whereby in the groups alkenyl, cycloalkenyl, alkynyl and substituted alkyl the carbon atom being in α-position to the unsaturation of the —CN group or aryl as defined above respectively, is connected with the carbon atoms of position 7 of the pyrimido[2,1-f]purine (I): reaction of a compound of formula XII or a sodium salt thereof, wherein $R^1$, $R^2$, $R^4$ and $R^5$ are defined as above, in the presence of sodium hydride in an aprotic organic solvent with a compound hal-$R^3$ (XIII), wherein hal is halogen,

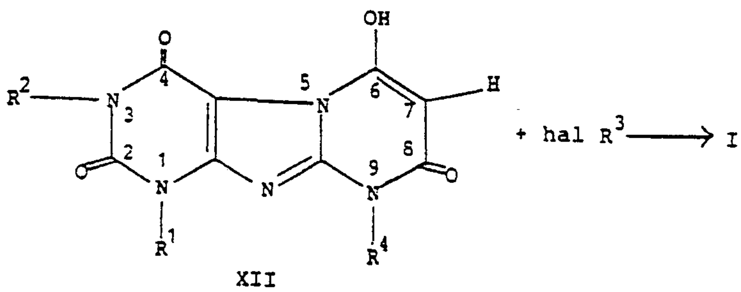

XIII

XII

whereby when $R^3$ is alkenyl, cycloalkenyl, alkynyl or substituted alkyl the halogen is in 2-position to the unsaturated of the —CN group or aryl as defined above respectively;

c) for the preparation of compounds of formula I, wherein $R^1$ and $R^2$ are the same or different and are hydrogen, alkyl having 1 to 8 carbon atoms, cycloalkyl having 4 to 8 carbon atoms, aryl as defined above;

alkyl (having 1 to 8 carbon atoms) substituted by cycloalkyl (having 4 to 8 carbon atoms), aryl as defined above or heterocyclic radical as defined above;

$R^3$ is alkyl containing 2 to 8 carbon atoms, cycloalkyl containing 5 to 8 carbon atoms;

$R^4$ is hydrogen, alkyl containing 1 to 8 carbon atoms, aryl as defined above, a heterocyclic radical as defined above;

alkyl (containing 1 to 8 carbon atoms) substituted by cycloalkyl (containing 4 to 8 carbon atoms), aryl as defined above or a heterocyclic radical as defined above;

$R^5$ is hydrogen, acyl containing 2 to 8 carbon atoms or alkyl containing 1 to 4 carbon atoms: hydrogenation of corresponding compounds of formula I, wherein $R^1$, $R^2$, $R^4$ and $R^5$ are defined as above and $R^3$ is alkenyl containing 2 to 8 carbon atoms, cycloalkenyl containing 5 to 8 carbon atoms or alkynyl containing 3 to 8 carbon atoms;

followed if desired by one or more aftertreatments

i) alkylation of compound of formula I wherein $R^5$ is hydrogen to form the corresponding compound wherein $R^5$ is alkyl;

ii) esterification of a compound of formula I, wherein $R^3$ is alkyl substituted by hydroxy to form the corresponding acyloxyalkyl-compound;

iii) salt formation;

iv) reduction of a compound of formula I, wherein $R^8$ is $-(CH_2)_rC(O)OR^8$ to the corresponding compound wherein $R^3$ is $-(CH_2)_rCH_2OH$.

2. Process according to claim 1a, characterized in that for the preparation of compounds (I) wherein $R^3$ is hydrogen, alkyl, cycloalkyl, alkyl substituted by cycloalkyl, and -alkyl-$C_nH_{2n+1}$, wherein X is $N-C_pH_{2p+1}$ the 8-aminoxanthine (II) is reacted with a diester of the $R^3$-containing malonic acidinthe presence of a base such as sodium methoxide at elevated temperature.

3. Process according to claim 1a, characterized in that for the preparation of compounds wherein $R^3$ is hydrogen, the 8-aminoxanthine (II) is reacted with a half-ester of the $R^3$-containing malonic acid halogenide in a suitable solvent at elevated temperature.

4. Process according to claim 1a, characterized in that the 8-aminoxanthine (II) is reacted with a di-ester of the $R^3$-containing malonic acid and a stoichiometric amount of a base (e.g. sodium hydride) in an organic solvent.

5. Process according to any one of claims 1 to 4, characterized in that a compound is prepared, wherein

$R^1$ and $R^2$ are the same or different and are alkyl having 1 to 8 carbon atoms, cycloalkyl having 3 to 8 carbon atoms, alkenyl having 3 to 8 carbon atoms, phenyl;

alkyl (having 1 to 8 carbon atoms) substituted by cycloalkyl (having 3 to 8 carbon atoms), phenyl, pyridyl or thienyl;

$R^3$ is hydrogen, alkyl containing 1 to 8 carbon atoms, cycloalkyl containing 3 to 8 carbon atoms, alkenyl containing 2 to 8 carbon atoms, cycloalkenyl containing 5 to 8 carbon atoms, alkynyl containing 3 to 8 carbon atoms, acyloxyalkyl containing 2 to 12 carbon atoms, phenyl;

alkyl (containing 1 to 8 carbon atoms) substituted by phenyl, hydroxy, cyano, halo, pyridyl or thienyl;

-alkyl-$X-C_nH_{2n+1}$ wherein alkyl contains 1 to 8 carbon atoms, n is an integer from 0 to 4 and X is O, S or $-N-C_nH_{2n+1}$ wherein p is an integer from 0 to 4;

$-(CH_2)_rC(O)OR^8$ (wherein $R^8$ is hydrogen or alkyl having from 1 to 8 carbon atoms and r is an integer from 0 to 6);

$R^4$ is alkyl containing 1 to 8 carbon atoms, phenyl, pyridyl or thienyl;

alkyl (containing 1 to 8 carbon atoms) substituted by cycloalkyl (containing 3 to 8 carbon atoms), phenyl, pyridyl or thienyl;

$R^5$ is hydrogen, acyl containing 2 to 8 carbon atoms or alkyl containing 1 to 4 carbon atoms.

6. Process according to any one of claims 1 to 5, wherein

$R^1$ and $R^2$ are the same or different and are alkyl having 1 to 4 carbon atoms;

$R^3$ is hydrogen, alkyl containing 1 to 5 carbon atoms, alkenyl containing 3 to 5 carbon atoms, cyclohexenyl, alkynyl containing 3 carbon atoms, acyloxyalkyl containing 4 carbon atoms, phenyl;

alkyl (containing 1 or 2 carbon atoms) substituted by phenyl, hydroxy or cyano;

$-CH_2-CH_2-N-(CH_3)_2$, $-CH_2C(O)OH$, $-CH_2C(O)OCH_3$, $-CH_2CH_2OCH_2CH_3$, $-CH_2CH_2SCH_2CH_3$ or $-CO_2C_2H_5$;

$R^4$ is alkyl containing 1 to 6 carbon atoms, phenyl or fluoro-substituted phenyl;

alkyl (containing 1 or 2 carbon atoms) substituted by cyclohexyl, phenyl (unsubstituted or substituted by fluoro, chloro, or methoxy), thienyl or pyridyl;

$R^5$ is hydrogen, acetyl or methyl.

7. Process according to any one of claims 1 to 6, characterized in that a compound is prepared, wherein $R^1$ and $R^2$ are as defined above, $R^3$ is n-propyl, n-butyl, or 3-methyl-2-butenyl, $R^4$ is benzyl or p-fluorobenzyl and $R^5$ is hydrogen.

8. Process according to any one of claims 1 to 7, characterized in that a compound is prepared, wherein $R^1$ and $R^2$ are methyl.

9. Process according to any one of claims 1 to 8, characterized in that a compound is prepared, wherein $R^5$ is replaced by $Na^+$.

10. Process according to any one of claims 1 to 8, characterized in that

9-benzyl-1,3-dimethyl-8-hydroxy-7-n-propyl-9*H*-6-oxopyrimido[2,1-f]purine-2,4-dione,

9-(4′-fluorobenzyl)-6-hydroxy-1,3-dimethyl-7-n-propyl-9*H*-8-oxo-pyrimidio[2,1-f]purine-2,4-dione,

9-benzyl-1,3-dimethyl-8-hydroxy-7-(n-butyl)-9*H*-6-oxo-pyrimido[2,1-f]purine-2,4-dione,

9-(4-fluorobenzyl)-1,3-dimethyl-6-hydroxy-7-(n-butyl)-9*H*-8-oxo-pyrimido[2,1-f]purine-2,4-dione,

9-benzyl-1,3-dimethyl-6-hydroxy-7-(3-methyl-2-butenyl)pyrimido[2,1-f]purine-2,4,8(1,*H*,3*H*,9*H*)trione

or

sodium salt of 1,3-dimethyl-9-(4-fluorobenzyl)-6-hydroxy-7-(3-methyl-2-butenyl)-pyrimido[2,1-f]-purine-2,4,8(1*H*,3*H*,9*H*)trione, is prepared.

**Patentansprüche für die Vertragsstaaten: BE CH DE FR GB IT LI, LU NL SE**

1. Verbindung der allgemeinen Formel (I)

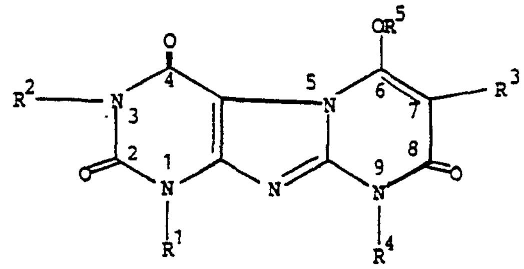

(I)

und ihr Tautomer und die pharmazeutisch annehmbaren Salze derselben, worin

$R^1$ und $R^2$ gleich oder verschieden sind und Wasserstoff, Alkyl mit 1 bis 8 Kohlenstoff-Atomen, Cycloalkyl mit 3 bis 8 Kohlenstoff-Atomen, Alkenyl mit 3 bis 8 Kohlenstoff-Atomen, Alkinyl mit 3 bis 8 Kohlenstoff-Atomen, Aryl wie Phenyl, Naphthyl, Phenanthryl oder substituiertes Phenyl, Alkyl (mit 1 bis 8 Kohlenstoff-Atomen), das durch Cycloalkyl (mit 3 bis 8 Kohlenstoff-Atomen), Aryl wie oben definiert oder einen aus Chinolinyl, Isochinolinyl, Pyridinyl, Thiazolyl, 1,3,4-Thiadiazolyl und Thiophenyl ausgewählten heterocyclischen Rest substituiert ist, sind,

wobei jeder der oben genannten substituierten Phenyl-Reste und substituierten heterocyclischen Reste durch ein bis drei Reste substituiert sein kann, die unabhängig ausgewählt sind aus Halogen (d.h. Fluoro, Chloro, Bromo und Iodo), Trifluoromethyl, Nitro, Cyano, $C_1$- bis $C_6$-Alkoxy, $C_1$- bis $C_6$-Alkyl, Phenyl, Hydroxy, $C_1$- bis $C_6$-Alkylthio,

$O^-$
|
$—S^+—(C_1$- bis $C_6$-Alkyl),

$—SO_2—(C_1$- bis $C_6$-Alkyl), $—O—(C_3$- bis $C_8$-Alkenyl), $—O—(C_3$- bis $C_8$-Alkinyl), $—OC_mH_{2m}$-phenyl, worin m eine ganze Zahl von 0 bis 4 ist, $C_1$- bis $C_6$-Acyloxy, Hydroxy—($C_1$- bis $C_6$-alkyl), ($C_1$- bis $C_6$-Acyloxy)-($C_1$- bis $C_6$-alkyl), Cyano—($C_1$- bis $C_6$-alkyl), $—CONH_2$, $—CO_2H$ und ($C_1$- bis $C_4$-Alkyl)—$CO_2$($C_1$- bis $C_4$-alkyl);

$R^3$ Wasserstoff, Alkyl mit 1 bis 8 Kohlenstoff-Atomen, Cycloalkyl mit 3 bis 8 Kohlenstoff-Atomen, Alkenyl mit 2 bis 8 Kohlenstoff-Atomen, Cycloalkylenyl mit 5 bis 8 Kohlenstoff-Atomen, Alkinyl mit 3 bis 8 Kohlenstoff-Atomen, Acyloxyalkyl mit 2 bis 12 Kohlenstoff-Atomen, Oxoalkyl mit 1 bis 8 Kohlenstoff-Atomen, Aryl, wie oben definiert wurde;

Alkyl (mit 1 bis 8 Kohlenstoff-Atomen), das durch Cycloalkyl (mit 3 bis 8 Kohlenstoff-Atomen), Cycloalkenyl (mit 5 bis 8 Kohlenstoff-Atomen), Aryl, wie oben definiert, Hydroxy, Cyano, Halogeno oder heterocyclische Reste, wie oben definiert, substituiert ist;

-alkyl—X—$C_nH_{2n+1}$, worin Alkyl 1 bis 8 Kohlenstoff-Atome enthält, n eine ganze Zahl von 0 bis 4 ist und X O, S, $S^+—O^-$, $SO_2$ oder —N—$C_pH_{2p+1}$ ist, worin p eine ganze Zahl von 0 bis 4 ist;

$—(CH_2)_qC(O)NR^6R^7$ (worin $R^6$ und $R^7$ unabhängig Wasserstoff oder Alkyl mit 1 bis 8 Kohlenstoff-Atomen sind und q eine ganze Zahl von 0 bis 6 ist),

$—(CH_2)_rC(O)OR^8$ worin $R^8$ Wasserstoff oder Alkyl mit 1 bis 8 Kohlenstoff-Atomen ist und r eine ganze Zahl von 0 bis 6 ist) ist;

$R^4$ Wasserstoff, Alkyl mit 1 bis 8 Kohlenstoff-Atomen, Aryl, wie oben definiert, ein heterocyclischer Rest, wie oben definiert;

Alkyl (mit 1 bis 8 Kohlenstoff-Atomen), das durch Cycloalkyl (mit 3 bis 8 Kohlenstoff-Atomen), Aryl, wie oben definiert, oder einen heterocyclischen Rest, wie oben definiert, substituiert ist, ist;

$R^5$ Wasserstoff Acyl mit 2 bis 8 Kohlenstoff-Atomen oder Alkyl mit 1 bis 4 Kohlenstoff-Atomen ist.

2. Verbindung nach Anspruch 1, dadurch gekennzeichnet, daß

$R^1$ und $R^2$ gleich oder verschieden sind und Alkyl mit 1 bis 8 Kohlenstoff-Atomen, Cycloalkyl mit 3 bis 8 Kohlenstoff-Atomen, Alkenyl mit 3 bis 8 Kohlenstoff-Atomen, Phenyl;

Alkyl (mit 1 bis 8 Kohlenstoff-Atomen), das durch Cycloalkyl (mit 3 bis 8 Kohlenstoff-Atomen), Phenyl, Pyridinyl, oder Thienyl, substituiert ist, sind,

$R^3$ Wasserstoff, Alkyl mit 1 bis 8 Kohlenstoff-Atomen, Cycloalkyl mit 3 bis 8 Kohlenstoff-Atomen, Alkenyl mit 2 bis 8 Kohlenstoff-Atomen, Cycloalkenyl mit 5 bis 8 Kohlenstoff-Atomen, Alkinyl mit 3 bis 8 Kohlenstoff-Atomen, Acyloxyalkyl mit 2 bis 12 Kohlenstoff-Atomen, Phenyl;

Alkyl (mit 1 bis 8 Kohlenstoff-Atomen), das durch Phenyl, Hydroxy, Cyano, Halogeno, Pyridyl oder Thienyl substituiert ist;

-alkyl—X—$C_nH_{2n+1}$, worin Alkyl 1 bis 8 Kohlenstoff-Atome enthält, n eine ganze Zahl von 0 bis 4 ist und X O, S oder —N—$C_pH_{2p+1}$ ist, worin p eine ganze Zahl von 0 bis 4 ist;

$—(CH_2)_rC(O)OR^8$ (worin $R^8$ Wasserstoff oder Alkyl mit 1 bis 8 Kohlenstoff-Atomen ist und r eine ganze Zahl von 0 bis 6 ist) ist;

$R^4$ Alkyl mit 1 bis 8 Kohlenstoff-Atomen, Phenyl, Pyridyl oder Thienyl;

Alkyl (mit 1 bis 8 Kohlenstoff-Atomen), das durch Cycloalkyl (mit 3 bis 8 Kohlenstoff-Atomen), Phenyl, Pyridyl, oder Thienyl substituiert ist, ist;

$R^5$ Wasserstoff, Acyl mit 2 bis 8 Kohlenstoff-Atomen oder Alkyl mit 1 bis 4 Kohlenstoff-Atomen ist.

3. Verbindung nach Ansprüchen 1 oder 2, dadurch gekennzeichnet, daß

$R^1$ und $R^2$ gleich oder verschieden sind und Alkyl mit 1 bis 4 Kohlenstoff-Atomen sind,

$R^3$ Wasserstoff, Alkyl mit 1 bis 5 Kohlenstoff-Atomen, Alkenyl mit 3 bis 5 Kohlenstoff-Atomen, Cyclohexenyl, Alkinyl mit 3 Kohlenstoff-Atomen, Acyloxyalkyl mit 4 Kohlenstoff-Atomen, Phenyl;

Alkyl (mit 1 oder 2 Kohlenstoff-Atomen), das durch Phenyl, Hydroxy oder Cyano substituiert ist;

$-CH_2-CH_2-N-(CH_3)_2$, $-CH_2C(O)OH$, $-CH_2C(O)OCH_3$, $-CH_2CH_2OCH_2CH_3$, $-CH_2CH_2SCH_2CH_3$ oder $-CO_2C_2H_5$ ist;

$R^4$ Alkyl mit 1 bis 6 Kohlenstoff-Atomen, Phenyl oder fluorosubstituiertes Phenyl;

Alkyl (mit 1 oder 2 Kohlenstoff-Atomen), das durch Cyclohexyl, Phenyl (unsubstituiert oder durch Fluoro, Chloro oder Methoxy substituiert), Thienyl oder Pyridyl ist;

$R^5$ Wasserstoff, Acetyl oder Methyl ist.

4. Verbindung nach irgendeinem der Ansprüche 1 bis 3, dadurch gekennzeichnet, daß $R^1$ und $R^2$ die oben angegebenen Bedeutungen haben, $R^3$ n-Propyl, n-Butyl oder 3-Methyl-2-butenyl ist, $R^4$ Benzyl oder p-Fluorobenzyl ist und $R^5$ Wasserstoff ist.

5. Verbindung nach irgendeinem der Ansprüche 1 bis 4, dadurch gekennzeichnet, daß $R^1$ und $R^2$ Methyl sind.

6. Verbindung nach irgendeinem der Ansprüche 1 bis 5 in Form ihres Natrium-Salzes, worin $R^5$ durch $Na^+$ ersetzt ist.

7. Verbindung nach irgendeinem der Ansprüche 1 bis 6, die

9-Benzyl-1,3-dimethyl-8-hydroxy-7-n-propyl-9*H*-6-oxopyrimido[2,1-f]purin-2,4-dion,

9-(4'-Fluorobenzyl)-6-hydroxy-1,3-dimethyl-7-n-propyl-9*H*-8-oxopyrimido[2,1-f]purin-2,4-dion,

9-Benzyl-1,3-dimethyl-8-hydroxy-7-(n-butyl)-9*H*-6-oxopyrimido[2,1-f]purin-2,4-dion,

9-(4-Fluorobenzyl)-1,3-dimethyl-6-hydroxy-7-(n-butyl)-9*H*-8-oxopyrimido[2,1-f]purin-2,4-dion,

9-Benzyl-1,3-dimethyl-6-hydroxy-7-(3-methyl-2-butenyl)-pyrimido[2,1-f]purin-2,4,8-(1*H*,3*H*,9*H*)trion oder

das Natrium-Salz von 1,3-Dimethyl-9-(4-fluorobenzyl)-6-hydroxy-7-(3-methyl-2-butenyl)-pyrimido[2,1-f]purin-2,4,8-(1*H*,3*H*,9*H*)trion ist.

8. Verfahren zur Herstellung von in irgendeinem der Ansprüche 1 bis 7 definierten Verbindungen, dadurch gekennzeichnet, daß die Verbindungen mittels eines geeigneten Verfahrens oder einer geeigneten Kombination von Verfahren hergestellt werden, die aus den folgenden Verfahren ausgewählt sind:

a) zur Herstellung von Verbindungen, wie sie in Anspruch 1 definiert sind, in denen $R^5$ Wasserstoff ist und $R^1$, $R^2$, $R^3$ und $R^4$ die in Anspruch 1 angegebenen Bedeutungen haben:

Reaktion eines 8-Aminoxanthins der Formel II

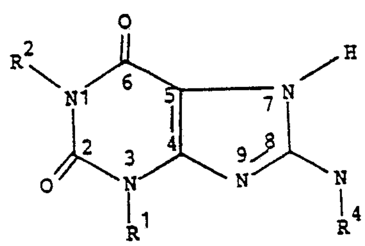

II

mit einem reaktionsfähigen Derivat einer $R^3$-substituierten Malonsäure;

b) zur Herstellung von Verbindungen der Formel I, in denen $R^1$, $R^2$ und $R^4$ die oben angegebenen Bedeutungen haben, $R^5$ Wasserstoff ist und

$R^3$ Methyl, Ethyl, Propyl, Alkenyl mit 3 bis 8 Kohlenstoff-Atomen, Cycloalkenyl mit 5 bis 8 Kohlenstoff-Atomen, Alkinyl mit 3 bis 8 Kohlenstoff-Atomen;

Alkyl (mit 1 bis 8 Kohlenstoff-Atomen), das durch Cyclopropyl, Cycloalkenyl (mit 5 bis 8 Kohlenstoff-Atomen, Aryl, wie oben definiert, oder Cyano; oder

$-(CH_2)-C(O)OR^8$ (worin $R^8$ Alkyl mit 1 bis 8 Kohlenstoff-Atomen ist) ist,

wobei in den Gruppen Alkenyl, Cycloalkenyl, Alkinyl und substituiertes Alkyl das Kohlenstoff-Atom in α-Position zu der Nichtsättigung oder der CN-Gruppe oder dem Aryl, wie jeweils oben definiert, mit dem Kohlenstoff-Atom der Position 7 des Pyrimido[2,1-f]purins (I) verbunden ist:

Reaktion einer Verbindung der Formel XII oder eines Natrium-Salzes derselben, worin $R^1$, $R^2$, $R^4$ und $R^5$ die oben angegebenen Bedeutungen haben, in Gegenwart von Natriumhydrid in einem aprotischen organischen Lösungsmittel mit einer Verbindung hal-$R^3$ (XIII), in der hal Halogen ist,

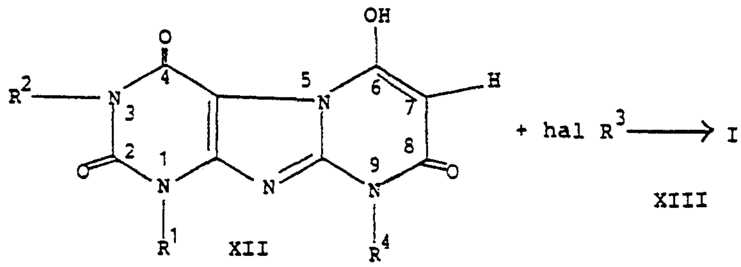

XII XIII

wobei, wenn $R^3$ Alkenyl, Cycloalkenyl, Alkinyl oder substituiertes Alkyl ist, das Halogen sich in der 2-Position zu der Nichtsättigung oder der CN-Gruppe oder dem Aryl, wie jeweils oben definiert, befindet;

c) zur Herstellung von Verbindungen der Formel I, in denen

$R^1$ und $R^2$ gleich oder verschieden sind und Wasserstoff, Alkyl mit 1 bis 8 Kohlenstoff-Atomen, Cycloalkyl mit 4 bis 8 Kohlenstoff-Atomen, Aryl, wie oben definiert;

Alkyl (mit 1 bis 8 Kohlenstoff-Atomen), das durch Cycloalkyl (mit 4 bis 8 Kohlenstoff-Atomen), Aryl, wie oben definiert, oder einen heterocyclischen Rest, wie oben definiert, substituiert ist, sind;

$R^3$ Alkyl mit 2 bis 8 Kohlenstoff-Atomen, Cycloalkyl mit 5 bis 8 Kohlenstoff-Atomen ist;

$R^4$ Wasserstoff, Alkyl mit 1 bis 8 Kohlenstoff-Atomen, Aryl, wie oben definiert, oder ein heterocyclischer Rest, wie oben definiert;

Alkyl (mit 1 bis 8 Kohlenstoff-Atomen), das durch Cycloalkyl (mit 4 bis 8 Kohlenstoff-Atomen), Aryl, wie oben definiert, oder einen heterocyclischen Rest, wie oben definiert, substituiert ist, ist;

$R^5$ Wasserstoff, Acyl mit 2 bis 8 Kohlenstoff-Atomen oder Alkyl mit 1 bis 4 Kohlenstoff-Atomen ist:

Hydrierung der entsprechenden Verbindungen der Formel I, in denen $R^1$, $R^2$, $R^4$ und $R^5$ die oben angegebenen Bedeutungen haben und $R^3$ Alkenyl mit 2 bis 8 Kohlenstoff-Atomen, Cycloalkenyl mit 5 bis 8 Kohlenstoff-Atomen oder Alkinyl mit 3 bis 8 Kohlenstoff-Atomen ist,

gewünschtenfalls gefolgt von einer oder mehreren Nachbehandlungen der

i) Alkylierung einer Verbindung der Formel I, in der $R^5$ Wasserstoff ist, unter Bildung der entsprechenden Verbindung, in der $R^5$ Alkyl ist;

ii) Veresterung einer Verbindung der Formel I, in der $R^3$ durch Hydroxy substituiertes Alkyl ist, unter Bildung der entsprechenden Acyloxyalkyl-Verbindung;

iii) Salz-Bildung;

iv) Reduktion einer Verbindung der Formel I, in der $R^7$ $-(CH_2)_rC(O)OR^8$ ist, zu der entsprechenden Verbindung, in der $R^3$ $-(CH_2)_rCH_2OH$ ist.

9. Verfahren nach Anspruch 8a, dadurch gekennzeichnet, daß zur Herstellung von Verbindungen (I); in denen $R^3$ Wasserstoff, Alkyl, Cycloalkyl, durch Cycloalkyl substituiertes Alkyl und -alkyl—X—$C_nH_{2n+1}$ ist, worin X —N—$C_pH_{2p+1}$ ist, das 8-Aminoxanthin (II) mit einem Diester der $R^3$ enthaltenden Malonsäure in Gegenwart einer Base wie Natriummethoxid bei erhöhter Temperatur umgesetzt wird.

10. Verfahren nach Anspruch 8a, dadurch gekennzeichnet, daß zur Herstellung von Verbindungen (I), in denen $R^3$ Wasserstoff ist, das 8-Aminoxanthin (II) mit einem Halbester des $R^3$ enthaltenden Malonsäurehalogenids in einem geeigneten Lösungsmittel bei erhöhter Temperatur umgesetzt wird.

11. Verfahren nach Anspruch 8a, dadurch gekennzeichnet, daß das 8-Aminoxanthin (II) mit einem Diester der $R^3$ enthaltenden Malonsäure und einer stöchiometrischen Menge einer Base (z.B. Natriumhydrid) in einem organischen Lösungsmittel umgesetzt wird.

12. Pharmazeutische Zusammensetzung, umfassend eine Verbindung, wie sie in irgendeinem der Ansprüche 1 bis 7 definiert ist oder mittels eines Verfahrens nach irgendeinem der Ansprüche 8 bis 11 erhalten worden ist, gewünschtenfalls in Form einer ausgeformten Einheitsdosis.

13. Verfahren zur Herstellung einer pharmazeutischen Zusammensetzung, dadurch gekennzeichnet, daß eine Verbindung, wie sie in irgendeinem der Ansprüche 1 bis 7 definiert ist oder mittels eines Verfahrens nach irgendeinem der Ansprüche 8 bis 11 erhalten worden ist, in eine für die therapeutische Verabreichung geeignete Form gebracht wird.

**Patentansprüche für den Vertragsstaate: AT**

1. Verfahren zur Herstellung von Verbindungen der allgemeinen Formel (I)

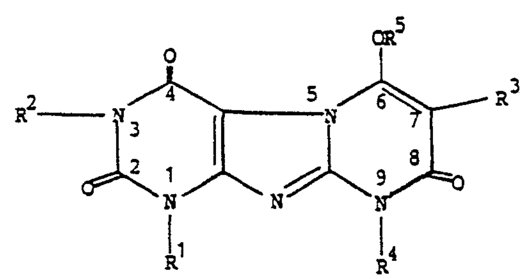

(I)

ihrer Tautomeren und ihrer pharmazeutisch annehmbaren Salze derselben, worin

$R^1$ und $R^2$ gleich oder verschieden sind und Wasserstoff, Alkyl mit 1 bis 8 Kohlenstoff-Atomen, Cycloalkyl mit 3 bis 8 Kohlenstoff-Atomen, Alkenyl mit 3 bis 8 Kohlenstoff-Atomen, Alkinyl mit 3 bis 8 Kohlenstoff-Atomen, Aryl wie Phenyl, Naphthyl, Phenanthryl oder substituiertes Phenyl, Alkyl (mit 1 bis 8 Kohlenstoff-Atomen), das durch Cycloalkyl (mit 3 bis 8 Kohlenstoff-Atomen), Aryl wie oben definiert oder einen aus Chinolinyl, Isochinolinyl, Pyridinyl, Thiazolyl, 1,3,4-Thiadiazolyl und Thiophenyl ausgewählten heterocyclischen Rest substituiert ist, sind,

wobei jeder der oben genannten substituierten Phenyl-Reste und substituierten heterocyclischen Reste durch ein bis drei Reste substituiert sein kann, die unabhängig ausgewählt sind aus Halogen (d.h. Fluoro, Chloro, Bromo und Iodo), Trifluoromethyl, Nitro, Cyano, $C_1$- bis $C_6$-Alkoxy, $C_1$- bis $C_6$-Alkyl, Phenyl, Hydroxy, $C_1$- bis $C_6$-Alkylthio,

$$\begin{array}{l} \quad O^- \\ \quad | \\ —S^+—(C_1\text{- bis } C_6\text{-Alkyl}), \end{array}$$

$—SO_2—(C_1$- bis $C_6$-Alkyl), $—O—(C_3$- bis $C_8$-Alkenyl), $—O—(C_3$- bis $C_8$-Alkinyl), $—OC_mH_{2m}$-phenyl, worin m eine ganze Zahl von 0 bis 4 ist, $C_1$- bis $C_6$-Acyloxy, Hydroxy—($C_1$- bis $C_6$-alkyl), ($C_1$- bis $C_6$-Acyloxy)-($C_1$- bis $C_6$-alkyl), Cyano—($C_1$- bis $C_6$-alkyl), $—CONH_2$, $—CO_2H$ und ($C_1$- bis $C_4$-Alkyl)$—CO_2$($C_1$- bis $C_4$-alkyl);

$R^3$ Wasserstoff, Alkyl mit 1 bis 8 Kohlenstoff-Atomen, Cycloalkyl mit 3 bis 8 Kohlenstoff-Atomen, Alkenyl mit 2 bis 8 Kohlenstoff-Atomen, Cycloalkylenyl mit 5 bis 8 Kohlenstoff-Atomen, Alkinyl mit 3 bis 8 Kohlenstoff-Atomen, Acyloxyalkyl mit 2 bis 12 Kohlenstoff-Atomen, Oxoalkyl mit 1 bis 8 Kohlenstoff-Atomen, Aryl, wie oben definiert wurde;

Alkyl (mit 1 bis 8 Kohlenstoff-Atomen), das durch Cycloalkyl (mit 3 bis 8 Kohlenstoff-Atomen), Cycloalkenyl (mit 5 bis 8 Kohlenstoff-Atomen), Aryl, wie oben definiert, Hydroxy, Cyano, Halogeno oder heterocyclische Reste, wie oben definiert, substituiert ist;

-alkyl$—X—C_nH_{2n+1}$, worin Alkyl 1 bis 8 Kohlenstoff-Atome enthält, n eine ganze Zahl von 0 bis 4 ist und X O, S, $S^+—O^-$, $SO_2$ oder $—N—C_pH_{2p+1}$ ist, worin p eine ganze Zahl von 0 bis 4 ist;

$—(CH_2)_qC(O)NR^6R^7$ (worin $R^6$ und $R^7$ unabhängig Wasserstoff oder Alkyl mit 1 bis 8 Kohlenstoff-Atomen sind und q eine ganze Zahl von 0 bis 6 ist),

$—(CH_2)_rC(O)OR^8$ worin $R^8$ Wasserstoff oder Alkyl mit 1 bis 8 Kohlenstoff-Atomen ist und r eine ganze Zahl von 0 bis 6 ist) ist;

$R^4$ Wasserstoff, Alkyl mit 1 bis 8 Kohlenstoff-Atomen, Aryl, wie oben definiert, ein heterocyclischer Rest, wie oben definiert;

Alkyl (mit 1 bis 8 Kohlenstoff-Atomen), das durch Cycloalkyl (mit 3 bis 8 Kohlenstoff-Atomen), Aryl, wie oben definiert, oder einen heterocyclischen Rest, wie oben definiert, substituiert ist, ist;.

$R^5$ Wasserstoff Acyl mit 2 bis 8 Kohlenstoff-Atomen oder Alkyl mit 1 bis 4 Kohlenstoff-Atomen ist, dadurch gekennzeichnet, daß die Verbindungen mittels eines geeigneten Verfahrens oder einer geeigneten Kombination von Verfahren hergestellt werden, die aus den folgenden Verfahren ausgewählt sind:

a) zur Herstellung von Verbindungen, wie sie in oben definiert sind, in denen $R^5$ Wasserstoff ist und $R^1$, $R^2$, $R^3$ und $R^4$ die oben angegebenen Bedeutungen haben:

Reaktion eines 8-Aminoxanthins der Formel II

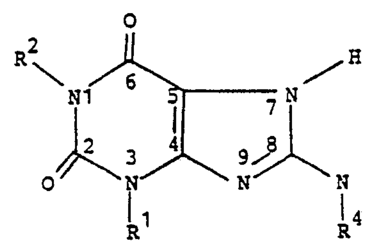

II

mit einem reaktionsfähigen Derivat einer $R^3$-substituierten Malonsäure;

b) zur Herstellung von Verbindungen der Formel I, in denen $R^1$, $R^2$ und $R^4$ die oben angegebenen Bedeutungen haben, $R^5$ Wasserstoff ist und

$R^3$ Methyl, Ethyl, Propyl, Alkenyl mit 3 bis 8 Kohlenstoff-Atomen, Cycloalkenyl mit 5 bis 8 Kohlenstoff-Atomen, Alkinyl mit 3 bis 8 Kohlenstoff-Atomen;

Alkyl (mit 1 bis 8 Kohlenstoff-Atomen), das durch Cyclopropyl, Cycloalkenyl (mit 5 bis 8 Kohlenstoff-Atomen, Aryl, wie oben definiert, oder Cyano; oder

$—(CH_2)—C(O)OR^8$ (worin $R^8$ Alkyl mit 1 bis 8 Kohlenstoff-Atomen ist) ist,

wobei in den Gruppen Alkenyl, Cycloalkenyl, Alkinyl und substituiertes Alkyl das Kohlenstoff-Atom in α-Position zu der Nichtsättigung oder der CN-Gruppe oder dem Aryl, wie jeweils oben definiert, mit dem Kohlenstoff-Atom der Position 7 des Pyrimido[2,1-f]purins (I) verbunden ist:

Reaktion einer Verbindung der Formel XII oder eines Natrium-Salzes derselben, worin $R^1$, $R^2$, $R^4$ und $R^5$ die oben angegebenen Bedeutungen haben, in Gegenwart von Natriumhydrid in einem aprotischen organischen Lösungsmittel mit einer Verbindung hal-$R^3$ (XIII), in der hal Halogen ist,

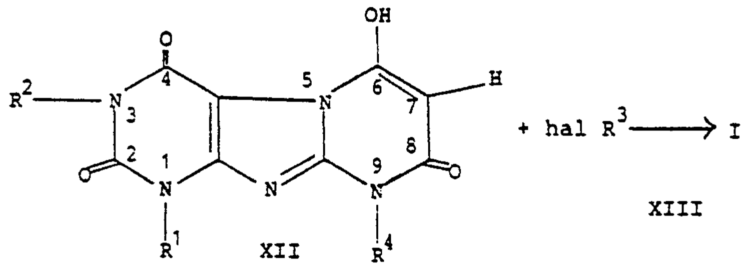

XIII

wobei, wenn $R^3$ Alkenyl, Cycloalkenyl, Alkinyl oder substituiertes Alkyl ist, das Halogen sich in der 2-Position zu der Nichtsättigung oder der CN-Gruppe oder dem Aryl, wie jeweils oben definiert, befindet;

c) zur Herstellung von Verbindungen der Formel I, in denen

$R^1$ und $R^2$ gleich oder verschieden sind und Wasserstoff, Alkyl mit 1 bis 8 Kohlenstoff-Atomen, Cycloalkyl mit 4 bis 8 Kohlenstoff-Atomen, Aryl, wie oben definiert;

Alkyl (mit 1 bis 8 Kohlenstoff-Atomen), das durch Cycloalkyl (mit 4 bis 8 Kohlenstoff-Atomen), Aryl, wie oben definiert, oder einen heterocyclischen Rest, wie oben definiert, substituiert ist, sind;

$R^3$ Alkyl mit 2 bis 8 Kohlenstoff-Atomen, Cycloalkyl mit 5 bis 8 Kohlenstoff-Atomen ist;

$R^4$ Wasserstoff, Alkyl mit 1 bis 8 Kohlenstoff-Atomen, Aryl, wie oben definiert, oder ein heterocyclischer Rest, wie oben definiert;

Alkyl (mit 1 bis 8 Kohlenstoff-Atomen), das durch Cycloalkyl (mit 4 bis 8 Kohlenstoff-Atomen), Aryl, wie oben definiert, oder einen heterocyclischen Rest, wie oben definiert, substituiert ist, ist;

$R^5$ Wasserstoff, Acyl mit 2 bis 8 Kohlenstoff-Atomen oder Alkyl mit 1 bis 4 Kohlenstoff-Atomen ist:

Hydrierung der entsprechenden Verbindungen der Formel I, in denen $R^1$, $R^2$, $R^4$ und $R^5$ die oben angegebenen Bedeutungen haben und $R^3$ Alkenyl mit 2 bis 8 Kohlenstoff-Atomen, Cycloalkenyl mit 5 bis 8 Kohlenstoff-Atomen oder Alkinyl mit 3 bis 8 Kohlenstoff-Atomen ist,

gewünschtenfalls gefolgt von einer oder mehreren Nachbehandlungen der

i) Alkylierung einer Verbindung der Formel I, in der $R^5$ Wasserstoff ist, unter Bildung der entsprechenden Verbindung, in der $R^5$ Alkyl ist;

ii) Veresterung einer Verbindung der Formel I, in der $R^3$ durch Hydroxy substituiertes Alkyl ist, unter Bildung der entsprechenden Acyloxyalkyl-Verbindung;

iii) Salz-Bildung;

iv) Reduktion einer Verbindung der Formel I, in der $R^7$ $-(CH_2)_rC(O)OR^8$ ist, zu der entsprechenden Verbindung, in der $R^3$ $-(CH_2)_rCH_2OH$ ist.

2. Verfahren nach Anspruch 1a, dadurch gekennzeichnet, daß zur Herstellung von Verbindungen (I); in denen $R^3$ Wasserstoff, Alkyl, Cycloalkyl, durch Cycloalkyl substituiertes Alkyl und -alkyl—X—$C_nH_{2n+1}$ ist, worin X —N—$C_pH_{2p+1}$ ist, das 8-Aminoxanthin (II) mit einem Diester der $R^3$ enthaltenden Malonsäure in Gegenwart einer Base wie Natriummethoxid bei erhöhter Temperatur umgesetzt wird.

3. Verfahren nach Anspruch 1a, dadurch gekennzeichnet, daß zur Herstellung von Verbindungen (I), in denen $R^3$ Wasserstoff ist, das 8-Aminoxanthin (II) mit einem Halbester des $R^3$ enthaltenden Malonsäurehalogenids in einem geeigneten Lösungsmittel bei erhöhter Temperatur umgesetzt wird.

4. Verfahren nach Anspruch 1a, dadurch gekennzeichnet, daß das 8-Aminoxanthin (II) mit einem Diester der $R^3$ enthaltenden Malonsäure und einer stöchiometrischen Menge einer Base (z.B. Natriumhydrid) in einem organischen Lösungsmittel umgesetzt wird.

5. Verfahren nach irgendeinem der Ansprüche 1 bis 4, dadurch gekennzeichnet, daß eine Verbindung hergestellt wird, in der

$R^1$ und $R^2$ gleich oder verschieden sind und Alkyl mit 1 bis 8 Kohlenstoff-Atomen, Cycloalkyl mit 3 bis 8 Kohlenstoff-Atomen, Alkenyl mit 3 bis 8 Kohlenstoff-Atomen, Phenyl;

Alkyl (mit 1 bis 8 Kohlenstoff-Atomen), das durch Cycloalkyl (mit 3 bis 8 Kohlenstoff-Atomen), Phenyl, Pyridinyl, oder Thienyl, substituiert ist, sind,

$R^3$ Wasserstoff, Alkyl mit 1 bis 8 Kohlenstoff-Atomen, Cycloalkyl mit 3 bis 8 Kohlenstoff-Atomen, Alkenyl mit 2 bis 8 Kohlenstoff-Atomen, Cycloalkenyl mit 5 bis 8 Kohlenstoff-Atomen, Alkinyl mit 3 bis 8 Kohlenstoff-Atomen, Acyloxyalkyl mit 2 bis 12 Kohlenstoff-Atomen, Phenyl;

Alkyl (mit 1 bis 8 Kohlenstoff-Atomen), das durch Phenyl, Hydroxy, Cyano, Halogeno, Pyridyl oder Thienyl substituiert ist;

-alkyl—X—$C_nH_{2n+1}$, worin Alkyl 1 bis 8 Kohlenstoff-Atome enthält, n eine ganze Zahl von 0 bis 4 ist und X O, S oder —N—$C_pH_{2p+1}$ ist, worin p eine ganze Zahl von 0 bis 4 ist;

$-(CH_2)_rC(O)OR^8$ (worin $R^8$ Wasserstoff oder Alkyl mit 1 bis 8 Kohlenstoff-Atomen ist und r eine ganze Zahl von 0 bis 6 ist) ist;

$R^4$ Alkyl mit 1 bis 8 Kohlenstoff-Atomen, Phenyl, Pyridyl oder Thienyl;

Alkyl (mit 1 bis 8 Kohlenstoff-Atomen), das durch Cycloalkyl (mit 3 bis 8 Kohlenstoff-Atomen), Phenyl, Pyridyl, oder Thienyl substituiert ist, ist;

$R^5$ Wasserstoff, Acyl mit 2 bis 8 Kohlenstoff-Atomen oder Alkyl mit 1 bis 4 Kohlenstoff-Atomen ist.

6. Verfahren nach irgendeinem der Ansprüche 1 bis 5, dadurch gekennzeichnet, daß

$R^1$ und $R^2$ gleich oder verschieden sind und Alkyl mit 1 bis 4 Kohlenstoff-Atomen sind,

$R^3$ Wasserstoff, Alkyl mit 1 bis 5 Kohlenstoff-Atomen, Alkenyl mit 3 bis 5 Kohlenstoff-Atomen, Cyclohexenyl, Alkinyl mit 3 Kohlenstoff-Atomen, Acyloxyalkyl mit 4 Kohlenstoff-Atomen, Phenyl;

Alkyl (mit 1 oder 2 Kohlenstoff-Atomen), das durch Phenyl, Hydroxy oder Cyano substituiert ist; $—CH_2—CH_2—N\text{-}(CH_3)_2$, $—CH_2C(O)OH$, $—CH_2C(O)OCH_3$, $—CH_2CH_2OCH_2CH_3$, $—CH_2CH_2SCH_2CH_3$ oder $—CO_2C_2H_5$ ist;

$R^4$ Alkyl mit 1 bis 6 Kohlenstoff-Atomen, Phenyl oder fluorosubstituiertes Phenyl;

Alkyl (mit 1 oder 2 Kohlenstoff-Atomen), das durch Cyclohexyl, Phenyl (unsubstituiert oder durch Fluoro, Chloro oder Methoxy substituiert), Thienyl oder Pyridyl ist;

$R^5$ Wasserstoff, Acetyl oder Methyl ist.

7. Verfahren nach irgendeinem der Ansprüche 1 bis 6, dadurch gekennzeichnet, daß eine Verbindung hergestellt wird, in der

$R^1$ und $R^2$ die oben angegebenen Bedeutungen haben, $R^3$ n-Propyl, n-Butyl oder 3-Methyl-2-butenyl ist, $R^4$ Benzyl oder p-Fluorobenzyl ist und $R^5$ Wasserstoff ist.

8. Verfahren nach irgendeinem der Ansprüche 1 bis 7, dadurch gekennzeichnet, daß keine Verbindung hergestellt wird, in der

$R^1$ und $R^2$ Methyl sind.

9. Verfahren nach irgendeinem der Ansprüche 1 bis 8, dadurch gekennzeichnet, daß eine Verbindung hergestellt wird, in der $R^5$ durch $Na^+$ ersetzt ist.

10. Verfahren nach irgendeinem der Ansprüche 1 bis 8, dadurch gekennzeichnet, daß

9-Benzyl-1,3-dimethyl-8-hydroxy-7-n-propyl-9*H*-6-oxopyrimido[2,1-f]purin-2,4-dion,

9-(4'-Fluorobenzyl)-6-hydroxy-1,3-dimethyl-7-n-propyl-9*H*-8-oxopyrimido[2,1-f]purin-2,4-dion,

9-Benzyl-1,3-dimethyl-8-hydroxy-7-(n-butyl)-9*H*-6-oxopyrimido[2,1-f]purin-2,4-dion,

9-(4-Fluorobenzyl)-1,3-dimethyl-8-hydroxy-7-(n-butyl)-9*H*-8-oxopyrimido[2,1-f]purin-2,4-dion,

9-Benzyl-1,3-dimethyl-6-hydroxy-7-(3-methyl-2-butenyl)-pyrimido[2,1-f]purin-2,4,8-(1*H*,3*H*,9*H*)trion oder

das Natrium-Salz von 1,3-Dimethyl-9-(4-fluorobenzyl)-6-hydroxy-7-(3-methyl-2-butenyl)-pyrimido[2,1-f]purin-2,4,8-(1*H*,3*H*,9*H*)trion hergestellt werden.

**Revendications pour les Etats contractants: BE CH DE FR GB IT LI LU NL SE**

1. Composé de formule générale (I)

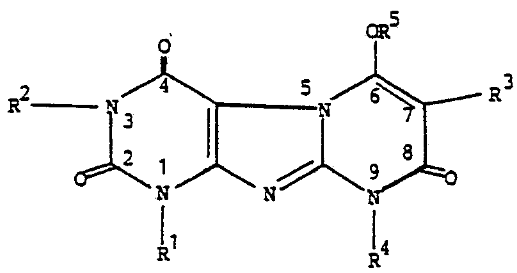

(I)

et son tautomère et ses sels acceptables en pharmacie.

dans lequel

$R^1$ et $R^2$ sont identiques ou différents et sont hydrogène, alcoyle ayant 1 à 8 atomes de carbone, cycloalcoyle ayant 3 à 8 atomes de carbone, alkényle ayant 3 à 8 atomes de carbone, alkynyle ayant 3 à 8 atomes de carbone, aryle comme phényle, naphtyle, phénanthryle ou phényle substitué;

alcoyle (ayant 1 à 8 atomes de carbone) substitué par cycloalcoyle (ayant 3 à 8 atomes de carbone), aryle tel que défini ci-dessus ou un radical hétérocyclique choisi parmi quinolinyle, isoquinolinyle, pyridinyle, thiazolyle, 1,3,4-thiadiazolyle et thiophényle;

chacun du phényle substitué et des radicaux hétérocycliques substitués ci-dessus peut être substitué par un à trois radicaux qui sont indépendamment choisis parmi halogène (c'est-à-dire fluoro, chloro, bromo, iodo), trifluorométhyle, nitro, cyano, alcoxy, $C_1$ à $C_6$, alcoyle $C_1$ à $C_6$, phényle, hydroxy, alkylthio $C_1$ à $C_6$,

$$\overset{O^-}{\overset{|}{—S^+}}\text{-(alcoyle } C_1 \text{ à } C_6),$$

$—SO_2$-(alcoyle $C_1$ à $C_6$), —O—(alkényle $C_3$ à $C_8$), —O—(alkynyle $C_3$ à $C_8$), $OC_mH_{2m}$-phényle où m est un nombre entier de 0 à 4, acyloxy $C_1$ à $C_6$, hydroxy-(alcoyle $C_1$ à $C_6$), (acyloxy $C_1$ à $C_6$)-(alcoyle $C_1$ à $C_6$)-, cyano-(alcoyle $C_1$ à $C_6$), $—CONH_2$, $—CO_2H$ et -(alcoyle $C_1$ à $C_4$)-$CO_2$(alcoyle $C_1$ à $C_4$);

$R^3$ est hydrogène, alcoyle contenant 1 à 8 atomes de carbone, cycloalcoyle contenant 3 à 8 atomes de carbone, alkényle contenant 2 à 8 atomes de carbone, cycloalkényle contenant 5 à 8 atomes de carbone,

alkynyle contenant 3 à 8 atomes de carbone, acyloxyalcoyle contenant 2 à 12 atomes de carbone, oxoalcoyle contenant 1 à 8 atomes de carbone, aryle tel que défini ci-dessus;

alcoyle (contenant 1 à 8 atomes de carbone) substitué par cycloalcoyle (contenant 3 à 8 atomes de carbone),

cycloalkényle (contenant 5 à 8 atomes de carbone), aryle tel que défini ci-dessus, hydroxy, cyano, halo ou radicaux hétérocycliques tels que définis ci-dessus;

-alkyl—X—$C_nH_{2n+1}$ où alcoyle contient 1 à 8 atomes de carbone, n est un nombre entier de 0 à 4 et X est O, S, $S^+$—$O^-$, $SO_2$ ou —N—$C_pH_{2p+1}$ où p est un nombre entier de 0 à 4;

—$(CH_2)_qC(O)NR^6R^7$ (où $R^6$ et $R^7$ sont indépendamment hydrogène ou alcoyle ayant de 1 à 8 atomes de carbone et q est un nombre entier de 0 à 6), —$(CH_2)_rC(O)OR^8$ (où $R^8$ est hydrogène ou alcoyle ayant de 1 à 8 atomes de carbone et r est un nombre entier de 0 à 6);

$R^4$ est hydrogène, alcoyle contenant 1 à 8 atomes de carbone, aryle tel que défini ci-dessus, un radical hétérocyclique tel que défini ci-dessus;

alcoyle (contenant 1 à 8 atomes de carbone) substitué par cycloalcoyle (contenant 3 à 8 atomes de carbone), aryle tel que défini ci-dessus ou un radical hétérocyclique tel que défini ci-dessus;

$R^5$ est hydrogène, acyle contenant 2 à 8 atomes de carbone ou alcoyle contenant 1 à 4 atomes de carbone.

2. Composé selon la revendication 1 où

$R^1$ et $R^2$ sont identiques ou différents et sont alcoyle ayant 1 à 8 atomes de carbone, cycloalcoyle ayant 3 à 8 atomes de carbone, alkényle ayant 3 à 8 atomes de carbone, phenyle;

alcoyle (ayant 1 à 8 atomes de carbone) substitué par cycloalcoyle (ayant 3 à 8 atomes de carbone), phényle, pyridyle ou thiényle;-

$R^3$ est hydrogène, alcoyle contenant 1 à 8 atomes de carbone, cycloalcoyle contenant 3 à 8 atomes de carbone, alkényle contenant 2 à 8 atomes de carbone, cycloalkényle contenant 5 à 8 atomes de carbone, alkynyle contenant 3 à 8 atomes de carbone, acyloxyalcoyle contenant 2 à 12 atomes de carbone, phényle;

alcoyle (contenant 1 à 8 atomes de carbone) substitué par phényle, hydroxy, cyano, halo, pyridyle ou thiényle;

-alkyl—X—$C_nH_{2n+1}$ où alcoyle contient 1 à 8 atomes de carbone, n est un nombre entier de 0 à 4 et X est O, S ou —N—$C_pH_{2p+1}$ où p est un nombre entier de 0 à 4;

—$(CH_2)_rC(O)OR^8$ (où $R^8$ est hydrogène ou alcoyle ayant de 1 à 8 atomes de carbone et r est un nombre entier de 0 à 6;

$R^4$ est alcoyle contenant 1 à 8 atomes de carbone, phényle, pyridyle ou thiényle;

alcoyle (contenant 1 à 8 atomes de carbone) substitué par cycloalcoyle (contenant 3 à 8 atomes de carbone), phényle, pyridyle ou thiényle;

$R^5$ est hydrogène, acyle contenant 2 à 8 atomes de carbone ou alcoyle contenant 1 à 4 atomes de carbone.

3. Composé selon la revendication 1 ou 2, où

$R^1$ et $R^2$ sont identiques ou différents et sont alcoyle ayant 1 à 4 atomes de carbone;

$R^3$ est hydrogène, alcoyle contenant 1 à 5 atomes de carbone, alkényle contenant 3 à 5 atomes de carbone, cyclohexényle, alkynyle contenant 3 atomes de carbone, acyloxyalcoyle contenant 4 atomes de carbone, phényle;

alcoyle (contenant 1 ou 2 atomes de carbone) substitué par phényle, hydroxy ou cyano;

—$CH_2$—$CH_2$—N—$(CH_3)_2$, —$CH_2C(O)OH$, —$CH_2C(O)OCH_3$, —$CH_2CH_2OCH_2CH_3$; —$CH_2CH_2SCH_2CH_3$ ou —$CO_2C_2H_5$;

$R^4$ est alcoyle contenant 1 à 6 atomes de carbone, phényle ou phényle fluoro-substitué;

alcoyle (contenant 1 ou 2 atomes de carbone) substitué par cyclohexyle, phényle (non substitué ou substitué par fluoro, chloro ou méthoxy), thiényle ou pyridyle;

$R^5$ est hydrogène, acétyle ou méthyle.

4. Composé selon l'une quelconque des revendications 1 à 3, où $R^1$ et $R^2$ sont tels que définis ci-dessus, $R^3$ est n-propyle, n-butyle ou 3-méthyl-2-butényle, $R^4$ est benzyle ou p-fluorobenzyle et $R^5$ est hydrogène.

5. Composé selon l'une quelconque des revendications 1 à 4, où $R^1$ et $R^2$ sont méthyle.

6. Composé selon l'une quelconque des revendications 1 à 5 sous la forme de son sel de sodium, où $R^5$ est remplacé par $Na^+$.

7. Composé selon l'une quelconque des revendications 1 à 6, qui est

9-benzyl-1,3-diméthyl-8-hydroxy-7-n-propyl-9*H*-6-oxo-pyrimido[2,1-f]purine-2,4-dione,

9-(4'-fluorobenzyl)-6-hydroxy-1,3-diméthyl-7-n-propyl-9*H*-8-oxo-pyrimido[2,1-f]purine-2,4-dione,

9-benzyl-1,3-diméthyl-8-hydroxy-7-(n-butyl)-9*H*-6-oxo-pyrimido[2,1-f]purine-2,4-dione,

9-(4-fluorobenzyl)-1,3-diméthyl-6-hydroxy-7-(n-butyl)-9*H*-8-oxopyrimido[2,1-f]purine-2,4-dione,

9-benzyl-1,3-diméthyl-6-hydroxy-7-(3-méthyl-2-butényl)-pyrimido[2,1-f]purine-2,4,8(1*H*,3*H*,9*H*)trione ou

sel de sodium de 1,3-dimethyl-9-(4-fluorobenzyl)-6-hydroxy-7-(3-méthyl-2-buténył)-pyrimido[2,1-f]purine-2,4,8(1*H*,3*H*,9*H*)trione.

8. Procédé pour la préparation de composés définis selon l'une quelconque des revendications 1 à 7, caractérisé en ce que les composés sont préparés par un procédé approprié ou combinaison de procédés choisis parmi les procédés suivants

a) pour la préparation de composés tels que définis à la revendication 1, où $R^5$ est hydrogène et $R^1$, $R^2$, $R^3$ et $R^4$ sont tels que définis à la revendication 1:

la réaction d'une 8-aminoxanthine de formule II

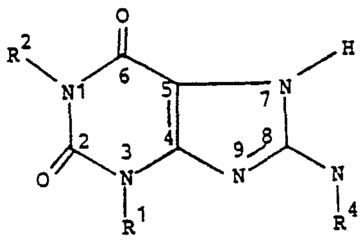

II

avec un dérivé réactif d'acide malonique $R^3$-substitué;

b) pour la préparation de composés de formule I, où $R^1$, $R^2$ et $R^4$ sont tels que définis ci-dessus, $R^5$ est hydrogène et $R^3$ est méthyle, éthyle, propyle, alkényle contenant 3 à 8 atomes de carbone, cycloalkényle contenant 5 à 8 atomes de carbone, alkynyle contenant 3 à 8 atomes de carbone;

alcoyle (contenant 1 à 8 atomes de carbone) substitué par cyclopropyle, cycloalkényle (contenant 5 à 8 atomes de carbone), aryle tel que défini ci-dessus ou cyano; ou

$—CH_2—C(O)OR^8$ (où $R^8$ est alcoyle ayant de 1 à 8 atomes de carbone);

dans les groupes alkényle, cycloalkényle, alkynyle et alcoyle substitué, l'atome de carbone étant en une position α relativement à l'insaturation ou au groupe —CN ou aryle tel que défini ci-dessus respectivement, est connecté à l'atome de carbone à la position 7 de la pyrimido[2,1,f]purine (I):

la réaction d'un composé de formule XII ou de son sel de sodium, où $R^1$, $R^2$, $R^4$ et $R^5$ sont tels que définis ci-dessus, en présence d'hydrure de sodium, dans un solvant organique aprotique avec un compose hal-$R^3$ (XIII), où hal est halogène,

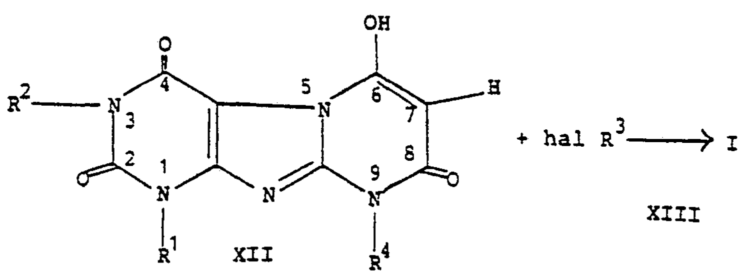

XII

XIII

quand $R^3$ est alkényle, cycloalkényle, alkynyle ou alcoyle substitué, l'halogène est en position 2 relativement à l'insaturation ou au groupe —CN ou aryle tel que défini ci-dessus respectivement;

c) pour la préparation de composés de formule I, où

$R^1$ et $R^2$ sont identiques ou différents et sont hydrogène, alcoyle ayant 1 à 8 atomes de carbone, cycloalcoyle ayant 4 à 8 atomes de carbone, aryle tel que défini ci-dessus;

alcoyle (ayant 1 à 8 atomes de carbone) substitué par cycloalcoyle (ayant 4 à 8 atomes de carbone), aryle tel que défini ci-dessus ou un radical hétérocyclique tel que défini ci-dessus;

$R^3$ est alcoyle contenant 2 à 8 atomes de carbone, cycloalcoyle contenant 5 à 8 atomes de carbone;

$R^4$ est hydrogène, alcoyle contenant 1 à 8 atomes de carbone, aryle tel que défini ci-dessus, un radical hétérocyclique tel que défini ci-dessus;

alcoyle (contenant 1 à 8 atomes de carbone) substitué par cycloalcoyle (contenant 4 à 8 atomes de carbone), aryle tel que défini ci-dessus ou un radical hétérocyclique tel que défini ci-dessus;

$R^5$ est hydrogène, acyle contenant 2 à 8 atomes de carbone ou alcoyle contenant 1 à 4 atomes de carbone:

l'hydrogénation de composés correspondants de formule I, où $R^1$, $R^2$, $R^4$ et $R^5$ sont tels que définis ci-dessus et $R^3$ est alkényle contenant 2 à 8 atomes de carbone, cycloalkényle contenant 5 à 8 atomes de carbone ou alkynyle contenant 3 à 8 atomes de carbone;

suivie, si on le souhaite, d'un ou plusieurs post-traitements

i) alcoylation du composé de formule I où $R^5$ est hydrogène pour former le composé correspondant où $R^5$ est alcoyle;

ii) estérification d'un composé de formule I, où $R^3$ est alcoyle substitué par hydroxy pour former le composé acyloxyalcoyle correspondant;

iii) formation de sel;

iv) réduction d'un composé de formule I, où $R^7$ est $—(CH_2)_rC(O)OR^8$ en composé correspondant où $R^3$ est $—(CH_2)_rCH_2OH$.

9. Procédé selon la revendication 8a, caractérisé en ce que pour la préparation des composés (I) où $R^3$ est hydrogène, alcoyle, cycloalcoyle, alcoyle substitué par cycloalcoyle et -alkyl$—X—C_nH_{2n+1}$, où X est $—N—C_pH_{2p+1}$, on fait réagir la 8-aminoxanthine (II) avec un diester d'acide malonique contenant $R^3$ en présence d'une base telle que le méthoxyde de sodium à température élevée.

10. Procédé selon la revendication 8a, caractérisé en ce que, pour la préparation des composés où $R^3$ est hydrogène, on fait réagir la 8-aminoxanthine (II) avec un hémiester de l'halogénure de l'acide malonqie contenant $R^3$ dans un solvant approprié à température élevée.

11. Procédé selon la revendication 8a, caractérisé en ce qu'on fait réagir la 8-aminoxanthine (II) avec un diester de l'acide malonique contenant $R^3$ et une quantité stoechiométrique d'une base (telle que l'hydrure de sodium) dans un solvant organique.

12. Composition pharmaceutique comprenant un composé tel que défini selon l'une quelconque des revendications 1 à 7 ou obtenu selon un procédé de l'une quelconque des revendications 8 à 11, si on le souhaite sous la forme d'une dose en forme.

13. Procédé de préparation d'une composition pharmaceutique caractérisé en ce qu'un composé tel que défini selon l'une quelconque des revendications 1 à 7 ou obtenu selon un procédé de l'une quelconque des revendications 8 à 11 est mis sous une forme appropriée à une administration thérapeutique.

**Revendications pour l'Etat contractant: AT**

1. Procédé pour la préparation de composés de formule générale (I)

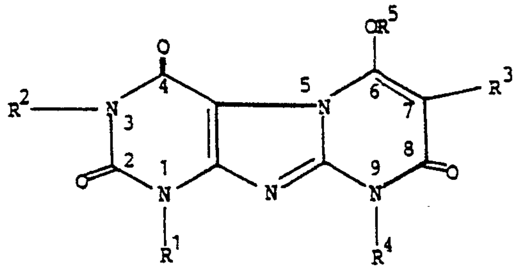

(I)

leurs tautoméres et leurs sels acceptables en pharmacie.
dans lequel
$R^1$ et $R^2$ sont identiques ou différents et sont hydrogène, alcoyle ayant 1 à 8 atomes de carbone, cycloalcoyle ayant 3 à 8 atomes de carbone, alkényle ayant 3 à 8 atomes de carbone, alkynyle ayant 3 à 8 atomes de carbone, aryle comme phényle, naphtyle, phénanthryle ou phényle substitué;

alcoyle (ayant 1 à 8 atomes de carbone) substitué par cycloalcoyle (ayant 3 à 8 atomes de carbone), aryle tel que défini ci-dessus ou un radical hétérocyclique choisi parmi quinolinyle, isoquinolinyle, pyridinyle, thiazolyle, 1,3,4-thiadiazolyle et thiophényle;

chacun du phényle substitué et des radicaux hétérocycliques substitués ci-dessus peut être substitué par un à trois radicaux qui sont indépendamment choisis parmi halogène (c'est-à-dire fluoro, chloro, bromo, iodo), trifluorométhyle, nitro, cyano, alcoxy, $C_1$ à $C_6$, alcoyle $C_1$ à $C_6$, phényle, hydroxy, alkylthio $C_1$ à $C_6$,

$O^-$
|
$—S^+$-(alcoyle $C_1$ à $C_6$),

$—SO_2$-(alcoyle $C_1$ à $C_6$), —O—(alkényle $C_3$ à $C_8$), —O—(alkynyle $C_3$ à $C_8$), $OC_mH_{2m}$-phényle où m est un nombre entier de 0 à 4, acyloxy $C_1$ à $C_6$, hydroxy-(alcoyle $C_1$ à $C_6$), (acyloxy $C_1$ à $C_6$)-(alcoyle $C_1$ à $C_6$)-, cyano-(alcoyle $C_1$ à $C_6$), $—CONH_2$, $—CO_2H$ et -(alcoyle $C_1$ à $C_4$)-$CO_2$(alcoyle $C_1$ à $C_4$);

$R^3$ est hydrogène, alcoyle contenant 1 à 8 atomes de carbone, cycloalcoyle contenant 3 à 8 atomes de carbone, alkényle contenant 2 à 8 atomes de carbone, cycloalkényle contenant 5 à 8 atomes de carbone, alkynyle contenant 3 à 8 atomes de carbone, acyloxyalcoyle contenant 2 à 12 atomes de carbone, oxoalcoyle contenant 1 à 8 atomes de carbone, aryle tel que défini ci-dessus;

alcoyle (contenant 1 à 8 atomes de carbone) substitué par cycloalcoyle (contenant 3 à 8 atomes de carbone),

cycloalkényle (contenant 5 à 8 atomes de carbone), aryle tel que défini ci-dessus, hydroxy, cyano, halo ou radicaux hétérocycliques tels que définis ci-dessus;

-alkyl—X—$C_nH_{2n+1}$ où alcoyle contient 1 à 8 atomes de carbone, n est un nombre entier de 0 à 4 et X est O, S, $S^+—O^-$, $SO_2$ ou —N—$C_pH_{2p+1}$ où p est un nombre entier de 0 à 4;

$—(CH_2)_qC(O)NR^6R^7$ (où $R^6$ et $R^7$ sont indépendamment hydrogène ou alcoyle ayant de 1 à 8 atomes de carbone et q est un nombre entier de 0 à 6), $—(CH_2)_rC(O)OR^8$ (où $R^8$ est hydrogène ou alcoyle ayant de 1 à 8 atomes de carbone et r est un nombre entier de 0 à 6);

$R^4$ est hydrogène, alcoyle contenant 1 à 8 atomes de carbone, aryle tel que défini ci-dessus, un radical hétérocyclique tel que défini ci-dessus;

alcoyle (contenant 1 à 8 atomes de carbone) substitué par cycloalcoyle (contenant 3 à 8 atomes de carbone), aryle tel que défini ci-dessus ou un radical hétérocyclique tel que défini ci-dessus;

$R^5$ est hydrogène, acyle contenant 2 à 8 atomes de carbone ou alcoyle contenant 1 à 4 atomes de carbone, caractérisé en ce que les composés sont préparés par un procédé approprié ou une combinaison de procédés choisis parmi les procédés suivants

a) pour la préparation de composés tels que définis ci-dessus, où $R^5$ est hydrogène et $R^1$, $R^2$, $R^3$ et $R^4$ sont tels que définis à la revendication 1:

la réaction d'une 8-aminoxanthine de formule II

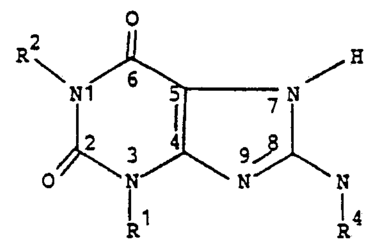

II

avec un dérivé réactif d'acide malonique $R^3$-substitué;

b) pour la préparation de composés de formule I, où $R^1$, $R^2$ et $R^4$ sont tels que définis ci-dessus, $R^5$ est hydrogène et $R^3$ est méthyle, éthyle, propyle, alkényle contenant 3 à 8 atomes de carbone, cycloalkényle contenant 5 à 8 atomes de carbone, alkynyle contenant 3 à 8.atomes de carbone;

alcoyle (contenant 1 à 8 atomes de carbone) substitué par cyclopropyle, cycloalkényle (contenant 5 à 8 atomes de carbone), aryle tel que défini ci-dessus ou cyano; ou

—$CH_2$—C(O)$OR^8$ (où $R^8$ est alcoyle ayant de 1 à 8 atomes de carbone);

dans les groupes alkényle, cycloalkényle, alkynyle et alcoyle substitué, l'atome de carbone étant en une position α relativement à l'insaturation ou au groupe —CN ou aryle tel que défini ci-dessus respectivement, est connecté à l'atome de carbone de la position 7 de la pyrimido[2,1,f]purine (I):

la réaction d'un composé de formule XII ou de son sel de sodium, où $R^1$, $R^2$, $R^4$ et $R^5$ sont tels que définis ci-dessus, en présence d'hydrure de sodium, dans un solvant organique aprotique avec un compose hal-$R^3$ (XIII), où hal est halogène,

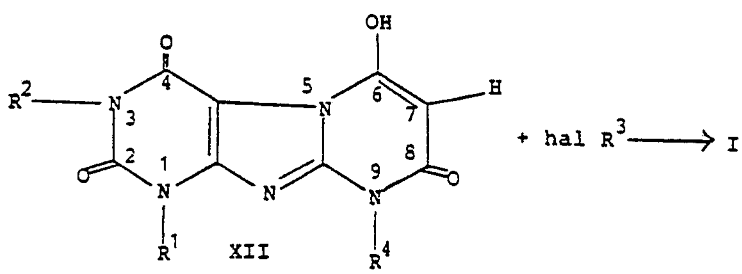

XIII

quand $R^3$ est alkényle, cycloalkényle, alkynyle ou alcoyle substitué, l'halogène est en position 2 relativement à l'insaturation ou au groupe —CN ou aryle tel que défini ci-dessus respectivement;

c) pour la préparation du composés de formule I, où

$R^1$ et $R^2$ sont identiques ou différents et sont hydrogène, alcoyle ayant 1 à 8 atomes de carbone, cycloalcoyle ayant 4 à 8 atomes de carbone, aryle tel que défini ci-dessus;

alcoyle (ayant 1 à 8 atomes de carbone) substitué par cycloalcoyle (ayant 4 à 8 atomes de carbone), aryle tel que défini ci-dessus ou un radical hétérocyclique tel que défini ci-dessus;

$R^3$ est alcoyle contenant 2 à 8 atomes de carbone, cycloalcoyle contenant 5 à 8 atomes de carbone;

$R^4$ est hydrogène, alcoyle contenant 1 à 8 atomes de carbone, aryle tel que défini ci-dessus, un radical hétérocyclique tel que défini ci-dessus;

alcoyle (contenant 1 à 8 atomes de carbone) substitué par cycloalcoyle (contenant 4 à 8 atomes de carbone), aryle tel que défini ci-dessus ou un radical hétérocyclique tel que défini ci-dessus;

$R^5$ est hydrogène, acyle contenant 2 à 8 atomes de carbone ou alcoyle contenant 1 à 4 atomes de carbone:

l'hydrogénation de composés correspondants de formule I, où $R^1$, $R^2$, $R^4$ et $R^5$ sont tels que définis ci-dessus et $R^3$ est alkényle contenant 2 à 8 atomes de carbone, cycloalkényle contenant 5 à 8 atomes de carbone ou alkynyle contenant 3 à 8 atomes de carbone;

suivie, si on le souhaite, d'un ou plusieurs post-traitements

i) alcoylation du composé de formule I où $R^5$ est hydrogène pour former le composé correspondant où $R^5$ est alcoyle;

ii) estérification d'un composé de formule I, où $R^3$ est alcoyle substitué par hydroxy pour former le composé acyloxyalcoyle correspondant;

iii) formation de sel;

iv) réduction d'un composé de formule I, où $R^7$ est —$(CH_2)_r$C(O)$OR^8$ en composé correspondant où $R^3$ est —$(CH_2)_rCH_2OH$.

2. Procédé selon la revendication 1a, caractérisé en ce que pour la préparation des composés (I) où $R^3$ est hydrogène, alcoyle, cycloalcoyle, alcoyle substitué par cycloalcoyle et -alkyl—X—$C_nH_{2n+1}$, où X est —N—$C_pH_{2p+1}$, on fait réagir la 8-aminoxanthine (II) avec un diester d'acide malonique contenant $R^3$ en présence d'une base telle que le méthoxyde de sodium à température élevée.

3. Procédé selon la revendication 1a, caractérisé en ce que, pour la préparation des composés où $R^3$ est hydrogène, on fait réagir la 8-aminoxanthine (II) avec un hémiester de l'halogénure de l'acide malonique contenant $R^3$ dans un solvant approprié à température élevée.

4. Procédé selon la revendication 1a, caractérisé en ce qu'on fait réagir la 8-aminoxanthine (II) avec un diester de l'acide malonique contenant $R^3$ et une quantité stoechiométrique d'une base (telle que l'hydrure de sodium) dans un solvant organique.

5. Procédé selon l'une quelconque des revendications 1 à 4, caractérisé en ce qu'un composé est préparé dans lequel

$R^1$ et $R^2$ sont identiques ou différents et sont alcoyle ayant 1 à 8 atomes de carbone, cycloalcoyle ayant 3 à 8 atomes de carbone, alkényle ayant 3 à 8 atomes de carbone, phenyle;

alcoyle (ayant 1 à 8 atomes de carbone) substitué par cycloalcoyle (ayant 3 à 8 atomes de carbone), phényle, pyridyle ou thiényle;

$R^3$ est hydrogène, alcoyle contenant 1 à 8 atomes de carbone, cycloalcoyle contenant 3 à 8 atomes de carbone, alkényle contenant 2 à 8 atomes de carbone, cycloalkényle contenant 5 à 8 atomes de carbone, alkynyle contenant 3 à 8 atomes de carbone, acyloxyalcoyle contenant 2 à 12 atomes de carbone, phényle;

alcoyle (contenant 1 à 8 atomes de carbone) substitué par phényle, hydroxy, cyano, halo, pyridyle ou thiényle;

-alkyl—X—$C_nH_{2n+1}$ où alcoyle contient 1 à 8 atomes de carbone, n est un nombre entier de 0 à 4 et X est O, S ou —N—$C_pH_{2p+1}$ où p est un nombre entier de 0 à 4;

—$(CH_2)_rC(O)OR^8$ (où $R^8$ est hydrogène ou alcoyle ayant de 1 à 8 atomes de carbone et r est un nombre entier de 0 à 6;

$R^4$ est alcoyle contenant 1 à 8 atomes de carbone, phényle, pyridyle ou thiényle;

alcoyle (contenant 1 à 8 atomes de carbone) substitué par cycloalcoyle (contenant 3 à 8 atomes de carbone), phényle, pyridyle ou thiényle;

$R^5$ est hydrogène, acyle contenant 2 à 8 atomes de carbone ou alcoyle contenant 1 à 4 atomes de carbone.

6. Procédé selon l'une quelconque des revendications 1 à 5, où

$R^1$ et $R^2$ sont identiques ou différents et sont alcoyle ayant 1 à 4 atomes de carbone;

$R^3$ est hydrogène, alcoyle contenant 1 à 5 atomes de carbone, alkényle contenant 3 à 5 atomes de carbone, cyclohexényle, alkynyle contenant 3 atomes de carbone, acyloxyalcoyle contenant 4 atomes de carbone, phényle;

alcoyle (contenant 1 ou 2 atomes de carbone) substitué par phényle, hydroxy ou cyano;
—$CH_2$—$CH_2$—N—$(CH_3)_2$, —$CH_2C(O)OH$, —$CH_2C(O)OCH_3$, —$CH_2CH_2OCH_2CH_3$, —$CH_2CH_2SCH_2CH_3$ ou —$CO_2C_2H_5$;

$R^4$ est alcoyle contenant 1 à 6 atomes de carbone, phényle ou phényle fluoro-substitué;

alcoyle (contenant 1 ou 2 atomes de carbone) substitué par cyclohexyle, phényle (non substitué ou substitué par fluoro, chloro ou méthoxy), thiényle ou pyridyle;

$R^5$ est hydrogène, acétyle ou méthyle.

7. Procédé selon l'une quelconque des revendications 1 à 6, caractérisé en ce qu'on prépare un composé dans lequel $R^1$ et $R^2$ sont tels que définis ci-dessus, $R^3$ est n-propyle, n-butyle ou 3-méthyl-2-butényle, $R^4$ est benzyle ou p-fluorobenzyle et $R^5$ est hydrogène.

8. Procédé selon l'une quelconque des revendications 1 à 7, caractérisé en ce qu'un composé est préparé dans lequel $R^1$ et $R^2$ sont méthyle.

9. Procédé selon l'une quelconque des revendications 1 à 8, caractérisé en ce qu'un composé est préparé dans lequel $R^3$ est remplacé par $Na^+$.

10. Procédé selon l'une quelconque des revendications 1 à 8 caractérisé en ce que l'on prépare
9-benzyl-1,3-diméthyl-8-hydroxy-7-n-propyl-9*H*-6-oxo-pyrimido[2,1-f]purine-2,4-dione,
9-(4′-fluorobenzyl)-6-hydroxy-1,3-diméthyl-7-n-propyl-9*H*-8-oxo-pyrimido[2,1-f]purine-2,4-dione,
9-benzyl-1,3-diméthyl-8-hydroxy-7-(n-butyl)-9*H*-6-oxo-pyrimido[2,1-f]purine-2,4-dione,
9-(4-fluorobenzyl)-1,3-diméthyl-6-hydroxy-7-(n-butyl)-9*H*-8-oxopyrimido[2,1-f]purine-2,4-dione,
9-benzyl-1,3-diméthyl-6-hydroxy-7-(3-méthyl-2-butényl)-pyrimido[2,1-f]purine-2,4,8(1*H*,3*H*,9*H*)trione
ou
sel de sodium de 1,3-diméthyl-9-(4-fluorobenzyl)-6-hydroxy-7-(3-méthyl-2-butényl)-pyrimido[2,1-f]purine-2,4,8(1*H*,3*H*,9*H*)trione.